# EUROPEAN PATENT SPECIFICATION

(11) **EP 4 590 851 B1**
(45) Date of publication and mention of the grant of the patent: **02.09.2026**
(21) Application number: 23776939.3
(22) Date of filing: 22.09.2023
(51) Int. Cl.: C12Q 1/6832, C12Q 1/6853, C12Q 1/6841

(54) **PADLOCK BLOCKING OLIGONUCLEOTIDE**
OLIGONUKLEOTID ZUR BLOCKIERUNG DES VORHÄNGESCHLOSSES
OLIGONUCLÉOTIDE DE BLOCAGE CADENAS

(30) Priority: 23.09.2022 GB 202213930
(43) Date of publication of application: 30.07.2025
(73) Proprietor: Navinci Diagnostics AB, 752 37 Uppsala (SE)
(72) Inventor: XU, Bo, 753 18 Uppsala (SE); LANDEGREN, Ulf, 751 08 Uppsala (SE)
(74) Representative: Dehns
(86) International application number: PCT/EP2023/076279
(87) International publication number: WO 2024/062116

(56) References cited:
- WO-A1-2014/076214
- WO-A1-2018/033528
- WO-A2-2015/083001
- GAO YANMIN ET AL: "Accurate genotyping of fragmented DNA using a toehold assisted padlock probe", BIOSENSORS AND BIOELECTRONICS, vol. 179, 1 May 2021 (2021-05-01), Amsterdam , NL, pages 113079, XP093341916, ISSN: 0956-5663, DOI: 10.1016/j.bios.2021.113079
- PEIDONG SHEN ET AL: "Multiplex target capture with double-stranded DNA probes", GENOME MEDICINE, vol. 5, no. 5, 1 January 2013 (2013-01-01), pages 50, XP055146836, ISSN: 1756-994X, DOI: 10.1186/gm454

## Description

### Field

The present invention relates to improvements in the use of padlock probes whereby the ligation of the padlock probe may be controlled, and in particular to a method of detecting a target nucleic acid in a sample which comprises the use of a padlock probe complexed with a blocking oligonucleotide. The blocking oligonucleotide binds the target-binding regions of the padlock probe and holds them apart in a manner which prevents their ligation, until the padlock probe is in the vicinity of the target nucleic acid molecule, at which point the padlock probe is released from the blocking probe so that it can bind its target, thereby reducing background signal (e.g. from non-specific ligation of the padlock probe). Also provided is a kit comprising a padlock probe and blocking oligonucleotide, which can be used in the methods of the invention.

### Background

The detection of target nucleic acid molecules has applications in many different fields, including notably clinically in the diagnosis, prognosis and/or treatment of disease, as well as in research and biosecurity. Nucleic acids are also frequently used as tags or labels in reporter systems for detecting other analytes, where the nucleic acid is detected as a proxy, or indicator for the analyte in question.

Target nucleic acid sequences may readily be detected using labelled hybridisation probes, but simple hybridisation probes have relatively high lower detection limits, and cannot readily be used to discriminate between similar nucleic acid sequences. To increase sensitivity, target nucleic acid molecules containing target sequences may typically be amplified, to increase the amount of target sequence available for detection. Rolling circle amplification (RCA) is commonly used for such amplification, and is typically used in conjunction with padlock probes.

RCA utilises a strand displacement polymerase enzyme, and requires a circular amplification template, which may be provided by a circularised padlock probe. Amplification of the circular template provides a concatenated RCA product, comprising multiple copies of a sequence complementary to that of the amplification template. Such a concatemer typically forms a ball or "blob", which may readily be visualised and detected, and thus RCA-based assays have been adopted for the detection of nucleic acids, and indeed, more generally, as reporter systems for the detection of any target analyte.

Padlock probes are typically linear oligonucleotides with two separate target-complementary binding regions, connected by an intervening "backbone" region. When the probe has bound (hybridised) to its target nucleic acid sequence, the ends of the probe may be ligated together to circularise the probe. The circularised padlock probe may then be used as the template for an RCA reaction, and the RCA product may be detected. This forms the basis of a number of detection assays in use today.

Padlock probes are highly specific, since they require dual recognition, or two binding sites for a target nucleic acid sequence. They further provide an extra layer of specificity, since only probes correctly base-paired at the ligation site will be ligated to generate the template for the molecule which will be detected. When the padlock probe hybridises to the target nucleic acid sequence with its target-binding regions directly adjacent to one another, the ends of the padlock probe may be ligated to each other directly. Alternatively, the target-binding regions of the padlock probe may hybridise to the target nucleic acid with a gap in between, and the gap may be filled in, either by hybridisation of one or more gap oligonucleotides in the gap region, or by a polymerase-catalysed extension of the hybridised 3 'end of the probe. In this way, the hybridised ends of the padlock probe may be ligated to each other indirectly, in that they are each hybridised to an intervening "gap sequence". Such "gap fill" padlock probes are also known as molecular inversion probes.

Generally speaking, in a detection method using padlock probes it is desired to remove unbound padlock probes, that is padlock probes which have not hybridised to their target nucleic acid (i.e. free padlock probes) or which have not become ligated, before the amplification (e.g. RCA) reaction is performed. In this way, mis-priming by free padlock probes present in the reaction mixture, for example by hybridising to the product of the RCA reaction, can be avoided. More particularly, free padlock probes which subsequently hybridise to the RCA product (RCP) generated in the RCA reaction can prime extension reactions templated by the RCP, leading to double-stranded products, to which detection oligonucleotides for detection of the RCP cannot bind. By removing, or "neutralising", such free padlocks such that they cannot participate in subsequent hybridisation or priming reactions, specificity and/or sensitivity of the method may be improved (in particular background can be reduced).

Current measures for removing free padlocks include washing. However, this requires a solid phase and thus is not applicable to homogenous, or so-called "in solution" methods, in which a solid phase is not used. Free padlocks may also be removed by enzymatic digestion, most notably using exonuclease enzymes which are able to digest nucleic acids with free, or unligated ends, but sparing circularised padlock probes, which do not have a free end. However, the disadvantage of this is not only the expense of an additional enzyme, but also the need to inactivate the exonuclease, so as to prevent the digestion of other assay/reaction components down the line (including the RCP, for example).

More generally, it is desirable to be able to control the ligation of padlock probes, to control the detection reaction, and ensure that the correct ligation reaction happens at the correct time. Other non-exonuclease based methods to avoid non-specific templated or non-templated ligation include the so-called "Unfold" approach (US 2014/0170654; Klaesson et al., 2018, Scientific Reports 8: 5400), wherein the ligation template for a padlock probe is hidden within a secondary structure such as a hairpin, such that it is not available to bind to the padlock probe, until it is released from the secondary structure by an enzymatic cleavage reaction which "unfolds", or opens, the hairpin. Indeed, in this technique the oligonucleotide(s) which form the padlock probe may also be released by an enzymatic "unfolding" reaction. The unfolding may be performed once reagents that have failed to bind their targets in the sample have been removed by washes. The SNAIL probe system (US 2019/0055594) uses ligation templates so short that they fail to stably bind the padlock probe partner, while upon proximal binding of the two members of the padlock probe/ligation template pair to the target nucleic acid molecule, the local concentration allows the padlock probe to interact with its template and undergo an enzymatic probe circularization reaction via ligation. The available approaches thus either involve a longer protocol and more complex reagents (the unfold approach) or sacrifice ligation efficiency by using a shorter ligation template (in the SNAIL approach the ligation template is 12 nt in length with, 6 nt hybridising nucleotide positions at each end of the padlock probe).

The present inventors have developed a new method to reduce non-specific templated and non-templated padlock probe ligation, thus reducing background from padlock probe-based nucleic acid detection.

Shen at al. "Multiplex target capture with double-stranded DNA probes." Genome Medicine vol. 5,5 50, 2013 discloses target capture using double-stranded probes, which consist of single-stranded, fully complementary long padlock probes (cLPPs).

Gao et al. "Accurate genotyping of fragmented DNA using a toehold assisted padlock probe." Biosensors & bioelectronics vol. 179 (2021): 113079 discloses a toehold-assisted padlock probe and toehold blocker, based on toehold-mediated strand displacement.

### Summary

The present method relies on a blocking oligonucleotide, which hybridises to the target-binding sites of the padlock probe and holds them in an unligatable position. The padlock probe is provided to the nucleic acid detection reaction in complex with the blocking oligonucleotide, and thus cannot bind its target until the blocking oligonucleotide is displaced or otherwise removed. The blocking oligonucleotide thus prevents ligation of padlock probes not bound to their target, reducing background signal.

Thus, in a first aspect provided herein is a method of detecting a target nucleic acid molecule in a sample, the method comprising:
(i) contacting the sample with a complex comprising a padlock probe and a blocking oligonucleotide,
   wherein the padlock probe comprises at its 5' and 3' ends target-binding regions which are complementary to probe-binding sites in the target nucleic acid molecule,
   and the blocking oligonucleotide comprises hybridisation sites which are complementary to the target-binding regions of the padlock probe;
   wherein the blocking oligonucleotide comprises a sequence which is not complementary to the padlock probe;
   wherein in the complex the hybridisation sites of the blocking oligonucleotide are hybridised to the target-binding regions of the padlock probe, in an arrangement whereby the 3' and 5' ends of the padlock probe cannot be ligated to each other;
(ii) causing removal of the blocking oligonucleotide from the complex such that the target-binding regions of the padlock probe hybridise to the probe-binding sites of the target nucleic acid molecule in an arrangement allowing direct or indirect ligation of the target-binding regions to each other;
(iii) directly or indirectly ligating the target-binding regions (or in other words, the 3' and 5' ends) of the padlock probe to each other, thereby circularising the padlock probe;
(iv) amplifying the circularised padlock probe or a part thereof; and
(v) detecting the amplification product of (iv) in order to detect the target nucleic acid sequence.

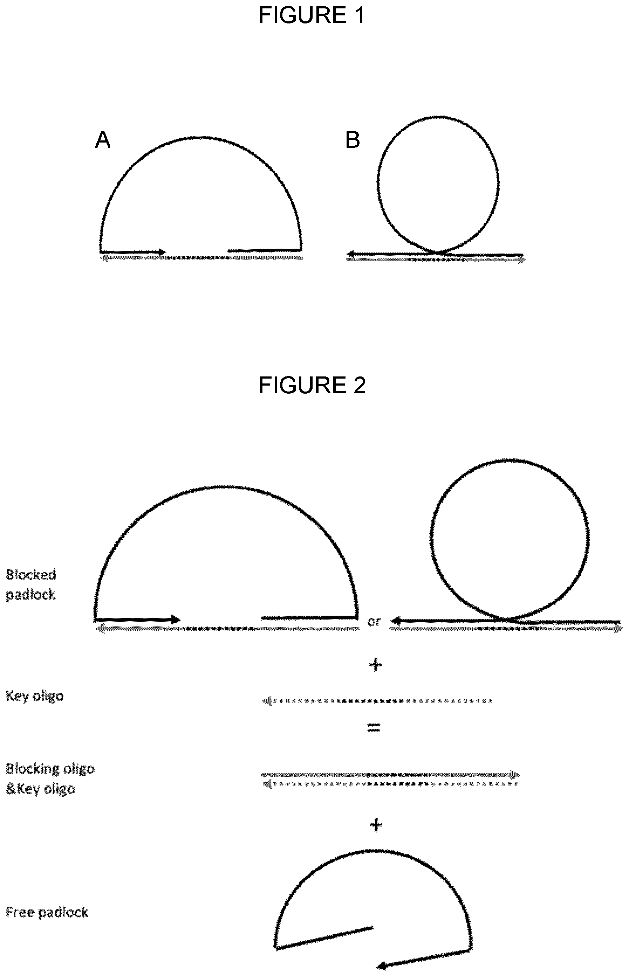

In one particular embodiment, as depicted in **Figure 1A** the 3' and 5' ends of the padlock probe hybridise to the blocking oligonucleotide with a gap between them, such that the ends are not adjacent to each other, and hence cannot be ligated to each other.

A short sequence, besides the blocking region, can be available on one end or both ends of the blocking oligonucleotide to serve as part of the hybridization region of a key oligonucleotide used to remove the blocking oligonucleotide.

In another embodiment, the 3' and 5' ends of the padlock probe are hybridised to the blocking oligonucleotide in opposing directions, i.e. such that they are oriented, or directed, away from one another, as depicted in **Figure 1B**. Also, in this arrangement the 3' and 5' ends do not lie adjacent to one another, and cannot be ligated together.

Thus, according to the present method, the padlock probe is hybridised to the blocking oligonucleotide in an arrangement wherein the 3' and 5' ends of the padlock probe are not in juxtaposition for direct ligation to one another.

The target nucleic acid molecule to be detected by the padlock probe may be a target analyte, that is the analyte which is the ultimate target, or entity, to be detected by the method. Alternatively, it may be generated from a target analyte, for example it may be a copy or amplicon of a target nucleic acid analyte. In a further embodiment, the target nucleic acid molecule may be a tag, or reporter (i.e. indicator) (or in another words, a nucleic acid label), for an analyte which is the subject of the assay method. The tag/reporter/label nucleic acid may be provided as part of a probe for detection of the target analyte, or it may be generated during the course of an assay for the target analyte, for example, by a ligation or extension or amplification or cleavage reaction etc. The target nucleic acid molecule may also be another nucleic acid molecule (e.g. oligonucleotide) used in the detection method, for example another probe, including another padlock probe.

More generally, the target nucleic acid molecule of the method is the target molecule of the padlock probe (i.e. the molecule targeted by the padlock probe), and may thus be seen as the ligation template for the padlock probe (more particularly, the intended, or target, ligation template). The method enables the hybridisation of the padlock probe to its intended target, and hence its ligation on that target, to be controlled.

In a particular embodiment, the detection method comprises a proximity assay, using proximity probes to detect a target analyte, and the target of the padlock probe is a nucleic acid domain of a proximity probe, or a product of the interaction of the nucleic acid domains of proximity probes (for example a ligation or extension product), or an oligonucleotide reagent (including another padlock probe), which is used in conjunction with the proximity probes.

Accordingly, in one particular embodiment, the method involves the use of proximity probes to detect a target analyte, wherein the proximity probes comprise a binding domain capable of binding directly or indirectly to a target analyte and a nucleic acid domain, and the padlock probe is capable of hybridising to the nucleic acid domain of a proximity probe, to a product of the interaction of the nucleic acid domains of the proximity probes, and/or to an oligonucleotide reagent which itself hybridises to the nucleic acid domain of a proximity probe e.g. to a padlock probe which is hybridised to the nucleic acid domain of a proximity probe.

In an embodiment, the target of the padlock probe is a nucleic acid domain of a proximity probe. In other words, the target binding regions of the padlock probe are capable of hybridising to (i.e. are complementary to) probe-binding sites in the nucleic acid domain of a proximity probe. Alternatively, or additionally, the padlock probe may be capable of hybridising to the nucleic acid domain of a proximity probe at a site outside the target binding regions of the padlock probe, for example at a site in the backbone region of the padlock probe.

In an embodiment, the padlock probe is hybridised at a site in its backbone region (that is the region between the two target-binding regions at the 3' and 5' ends of the padlock probe) to the nucleic acid domain of a proximity probe, and the target binding regions of the padlock probe are capable of hybridising to the nucleic acid domain of another proximity probe, or to an oligonucleotide (e.g. a padlock probe) which is hybridised to the nucleic acid domain of another proximity probe. In other words, the padlock probe may mediate the interaction between the nucleic acid domains of two proximity probes (i.e. of a proximity probe pair).

In an embodiment, the method herein is a method for detecting a target analyte in a sample, wherein the sample is contacted with first and second proximity probes for detection of the target analyte, each proximity probe being capable of binding directly or indirectly to the target analyte (i.e. each proximity probe is specific for the target analyte) and each comprising a nucleic acid domain, and wherein the nucleic acid domain of one proximity probe is hybridised to a padlock probe, and the target binding regions of the padlock probe are capable of hybridising to probe-binding sites located in either the nucleic acid domain of the other proximity probe, or in an oligonucleotide (e.g. a padlock probe) hybridised to the nucleic acid domain of the other proximity probe.

In accordance with the general principles of the method, the padlock probe is hybridised to the blocking oligonucleotide, and this is removed to allow the target-binding regions of the padlock probe to bind to the probe-binding sites in the nucleic acid domain of the other proximity probe.

This method can in a particular embodiment be used in the context of proximity probing to detect an interaction between two target molecules. In this embodiment, the method can be seen as a method for detecting an interaction between two target molecules in the sample, wherein the sample is contacted with a first proximity probe specific for the first target molecule and a second proximity probe specific for the second target molecule, each proximity probe comprising a nucleic acid domain,
wherein the nucleic acid domain of at least one proximity probe is hybridised to a padlock probe-blocking oligonucleotide complex,
and the target-binding regions of the padlock probe hybridise to probe-binding sites located in either the other nucleic acid domain or to another padlock probe hybridised to the other nucleic acid domain.

In another aspect, provided herein is a kit for detecting a target nucleic acid molecule in a sample, comprising a padlock probe and a blocking oligonucleotide as defined above.

### Detailed Description

The present method provides an improved method for detecting a target nucleic acid molecule in a sample. The method is based on detecting the target nucleic acid using a padlock probe. The padlock probe is provided to the detection reaction in complex with a blocking oligonucleotide, which prevents non-specific templated or non-templated ligation of the padlock probe, thereby reducing background signal. The blocking oligonucleotide occupies the target binding regions of the padlock probe, such that they are not available to hybridise elsewhere, and be ligated, until the blocking oligonucleotide is removed.

At its most general, the method is for detecting a target nucleic acid molecule (i.e. a nucleic acid molecule of interest). More particularly the method is for detecting a target nucleic acid sequence within a target nucleic acid molecule. The term "detecting" is used broadly herein to include any means of determining the presence of the target nucleic acid molecule. In the present method, commonly the ligated padlock probe is amplified, typically by RCA, and the target nucleic acid molecule is detected by detecting the presence or amount of the amplification product (e.g. RCP) which is generated, and can include detecting simply if it is present or not, or any form of measurement of the amplicon (e.g. RCP). Thus, the RCA product or other amplicon generated may be detected as the "signal" for the target nucleic acid molecule.

The presence of an amplification product in this case (i.e. the confirmation of its presence or amount) may be indicative or identificatory of the presence of the target nucleic acid molecule, as the successful generation of the amplification product is dependent on the presence of the target nucleic acid molecule (or more particularly the presence of a target nucleic acid sequence therein). Alternatively, when the padlock probe is used in the context of a proximity detection reaction (particularly for detecting the interaction of a pair of target molecules), the presence of an amplification product may be indicative or identificatory of the proximity probe target, e.g. the interaction between a pair of target molecules.

Quantitative and qualitative determinations, measurements or assessments are included in the term "determined", including semi-quantitative determination. Such determinations, measurements or assessments may be relative, for example when two or more different target nucleic acid sequences, or target molecules, in a sample are being detected, or absolute. Accordingly, in an embodiment the method may be for quantifying or determining the amount of target nucleic acid molecule or sequence which is present. The term "quantifying" when used in the context of quantifying a target nucleic acid molecule(s) or sequence(s) in a sample can refer to absolute or to relative quantification. Absolute quantification may be accomplished by inclusion of known concentration(s) of one or more control nucleic acid molecules and/or referencing the detected level of the target nucleic acid molecule or sequence with known control nucleic acid molecules or sequences (e.g. through generation of a standard curve). Alternatively, relative quantification can be accomplished by comparison of detected levels or amounts between two or more different target nucleic acid molecules, or different target sequences, to provide a relative quantification of each of the two or more different nucleic acid molecules or sequences, i.e., relative to each other. Thus, as noted above, ratios of target nucleic acid molecules or sequences present in sample may be determined. When the target nucleic acid molecules are representative of other target molecules, e.g. proteins, quantitative determination of the amount of target nucleic acid present in the sample may enable quantitation of the amount of the other target molecules (e.g. proteins) in the sample.

The target nucleic acid molecule is any nucleic acid molecule it is desired to detect by means of the padlock probe. It may be the ultimate target of an assay method (i.e. it may be a target analyte) but need not necessarily be so, and as indicated above it may be a nucleic acid molecule used or generated in an assay method. More particularly, the target may be a target sequence present in the nucleic acid molecule. It may in some embodiments be desirable to detect two or more target sequences which are present in a target molecule. In other embodiments, two or more target molecules, or two or more target sequences present in two or more target molecules may be detected. The method may be performed in multiplex to detect two or more different target sequences, for example, in one or more target nucleic acid molecules, and/or a target sequence in two or more target molecules. Thus, to detect target sequences in two or more different target molecules, a multiplicity of padlock probes may be used, each specific for a different target sequence, that is having target-binding regions which are complementary to binding sites in the target sequence, or which for example flank the different target sequences. It will be understood in this respect that where the target binding sites flank a target sequence in the molecule, the flanking binding sites in the different target molecules may be different for different target sequences, to allow for specific binding of the padlock probes. Thus, in such a situation the flanking sequences which are targeted by the padlock probes are discriminatory between different targets. They may thus be regarded as part of the target sequence. Alternatively and/or additionally, different and separate target sequences in the same target molecule may be detected, for example, different sequences in different genes on a chromosome, again using a multiplicity of different padlock probes, each specific for a different target sequence.

In a particular embodiment, the method is useful for detecting which of a number of possible different variant sequences is present in a given target molecule, for example whether a wild-type or mutant sequence is present, or which of a number of possible mutants, or different allelic variants, or polymorphisms etc. In such a protocol, the padlock probes may comprise target binding regions which are designed to discriminate between different variants. Alternatively, a gap-fill padlock probe may be used which hybridizes to flanking regions which flank all or multiple variants, and a gap-fill extension step is carried out, which generates a complementary copy of the variant sequence(s) that is (are) present. The nature of the variant sequence may be determined, or the variant may be detected, by detecting the complement of the variant sequence that is present in an amplification product, e.g. RCP or PCR product. This may be for example by sequencing, or by using a detection oligonucleotide which is specific for the variant sequence.

The method may thus be used to detect multiple target molecules or target sequences. The term "multiple" as used herein means two or more, for example, 3, 4, 5, 6, 8, 9, 10, 15, 20, 25, 30, 40, 50, 60, 70, 80, 90, or 100 or more. Indeed, thousands or tens of thousands of padlock probes may be used. The number of padlocks that can be used is not restricted, and can be varied. This will depend on the purpose of the method, the nature of the sample, and the target nucleic acid sequences to be detected, number of possible variants etc. Thus, to detect wild-type and mutant variants for example, the number of different padlocks will depend on the number of different mutants possible, and may be for example, 2-6, 2-5, 2-4 or 2-3 different padlocks. It will be appreciated that different aspects may be combined, in order to increase the overall multiplex of the assay. For example, in a given sample, the method may be used to detect different variants of different target sequences.

The target nucleic acid molecule or sequence may be any molecule or sequence it is desired to detect or identify. It may be DNA or RNA, or a modified variant thereof. Thus, the nucleic acid may be made up of ribonucleotides and/or deoxyribonucleotides as well as synthetic nucleotides that are capable of participating in Watson-Crick type or analogous base pair interactions. Thus, the nucleic acid may be, or may comprise, e.g. bi-sulphite converted DNA, LNA, PNA or any other derivative containing a non-nucleotide backbone.

As noted above, the target molecule or sequence may be an analyte it is desired to detect, for example a nucleic acid present in a sample, e.g. in a cell or tissue sample, or any biological or clinical sample etc. It may be a viral nucleic acid. Thus, it may be a naturally occurring sequence, or a derivative or copy or amplicon thereof. However, the method is not restricted to this, and the target molecule may instead be a reporter (or in other words, an indicator or proxy) for an analyte of an assay. Reporter nucleic acids may be used or generated in the course of an assay for any analyte, for example a protein or other biological molecule, or small molecule, in a sample. Thus, a reporter nucleic acid may be provided as a tag, or label, for a binding probe for an analyte, and may be detected in order to detect the analyte, for example in an immunoassay, e.g. as in an immunoPCR or immunoRCA reaction. A reporter nucleic acid may be generated in the course of an assay, for example by a ligation reaction in a proximity ligation assay, or an extension reaction in a proximity extension assay, or by a cleavage reaction, or such like, or indeed by any reaction employed in a detection method using nucleic acids, including for example as part of a signal amplification system. It can for example be generated by an amplification reaction. Such a reporter target nucleic acid may therefore be a synthetic or artificial sequence.

In an embodiment, the target nucleic acid is a DNA molecule, natural or synthetic. The target nucleic acid molecule may be coding or non-coding DNA, for example genomic DNA or a sub-fraction thereof, or may be derived from genomic DNA, e.g. a copy or amplicon thereof, or it may be cDNA or a sub-fraction thereof, or an amplicon or copy thereof etc.

In another embodiment, the target nucleic acid molecule is a target RNA molecule. It may be an RNA molecule in a pool of RNA or other nucleic acid molecules for example genomic nucleic acids, whether human or from any source, from a transcriptome, or any other nucleic acid (e.g. organelle nucleic acids, e.g. mitochondrial or plastid nucleic acids, or viral nucleic acids), whether naturally occurring or synthetic. The target RNA molecule may thus be or may be derived from coding (i.e. pre-mRNA or mRNA) or non-coding RNA sequences (such as tRNA, rRNA, snoRNA, miRNA, siRNA, snRNA, exRNA, piRNA and long ncRNA). In one embodiment, the target nucleic acid molecule is a micro RNA (miRNA). In one embodiment, the target RNA molecule is 16S RNA, for example wherein the 16S RNA is from and identificatory of a microorganism (e.g. a pathogenic microorganism) in a sample. Alternatively, the target RNA molecule may be genomic RNA, e.g. ssRNA or dsRNA of a virus having RNA as its genetic material. Notable such viruses include Ebola, HIV, SARS, SARS-CoV2, influenza, hepatitis C, West Nile fever, polio and measles. Accordingly, the target RNA molecule may be positive sense RNA, negative sense RNA, or double-stranded RNA from a viral genome, or positive-sense RNA from a retroviral RNA genome.

Where the target molecule is an RNA molecule, the method may comprise a preliminary step of generating a cDNA copy of the target RNA molecule. The cDNA molecule is then contacted with the padlock probe in step (i). Alternatively, a target RNA molecule may directly be contacted with the padlock probe. In other words, the first padlock probe may bind directly to the target RNA molecule.

The term "contacting" is used broadly herein to include any means of bringing the sample, or more particularly, the target nucleic acid molecule, into contact with the padlock probe/blocking oligonucleotide complex (referred to hereafter as "the complex"). This may involve for example, adding the complex to the sample, and holding, e.g. incubating, the resulting reaction mixture under conditions which allow the probe to hybridise to the target nucleic acid molecule. Alternatively, the sample, or an aliquot or portion thereof, may be added to the padlock probe, or to a reaction mix comprising the complex.

The sample on which the method of the invention is performed may be any biological sample, e.g. a research sample or a clinical sample. The method of the invention may thus be used as a research tool or a diagnostic tool. The sample may be any type of biological sample, e.g. a cell or tissue sample, a fluid sample, a cell lysate, etc. The sample may contain any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including blue green algae, fungi, bacteria, protozoa etc., or a virus. The cells may be for example human cells, avian cells, reptile cells etc., without limitation.

Representative samples thus include whole blood and blood-derived products such as plasma, serum and buffy coat, blood cells, urine, faeces, cerebrospinal fluid or any other body fluids (e.g. respiratory secretions, saliva, milk, etc.), tissues, biopsies, cell cultures, cell suspensions, conditioned media or other samples of cell culture constituents, etc. The method finds particular utility in histology and analysis of tissue and cell samples.

The sample may be freshly prepared, or may be pre-treated in any convenient or desired way to prepare for use in the method, for example by cell lysis or purification, isolation of the nucleic acid, etc. The sample may thus be processed as necessary, e.g. cells or tissue may, as required depending on the target molecules and other relevant factors, be fixed or permeabilised. Accordingly, fresh, frozen or fixed cells or tissues may be used, e.g. FFPE tissue (Formalin Fixed Paraffin Embedded). The present method is particularly advantageous as it is able to detect target molecules, and an interaction between them, at very low concentration.

The method may be a homogenous method, that is a method performed in solution, or it a may a heterogenous method, that is a method performed on or using a solid phase. This may depend on the sample which is used, and/or the target molecule or analyte it is desired to detect. For example, the method may be performed on cell or tissue samples for *in situ* detection of a target analyte, for example using proximity probe. This is desirable in instances of localised detection. Alternatively, the for other samples or detection assays, the method may be performed in solution, for example with liquid samples, e.g. plasma or serum samples etc., or processed sample, or preparations of target nucleic acids (e.g. amplicons etc.).

The sample analysed according to the present method is thus first contacted with a complex comprising a padlock probe and a blocking oligonucleotide. A padlock probe may be defined as a circularisable probe. The use of padlock or circularisable probes is well known in the art, including in the context of RCA reactions. A circularisable probe comprises one or more linear oligonucleotides which may be ligated together to form a circle. Padlock probes are well known and widely used and are well-reported and described in the literature. Thus, the principles of padlock probing are well understood and the design and use of padlock probes is known and described in the art. A padlock probe is typically a linear circularizable oligonucleotide which hybridizes to its target nucleic acid sequence or molecule in a manner which brings 5' and 3' ligatable ends of the probe into juxtaposition for ligation together, either directly, or as described above, indirectly, with a gap in between. By ligating the hybridised 5' and 3' ends of the probe, the probe is circularized. It is understood that for circularization (ligation) to occur, the ligatable 5' end of the padlock probe has a free 5' phosphate group.

To allow the juxtaposition of the ends of the padlock probe for ligation, the padlock probe is designed to have target-binding sites at its 5' and 3' ends. That is, the regions of complementarity which allow binding of the padlock probe to its target lie at the ends of the padlock probe. The target-binding sites (or target-binding regions) are complementary to probe-binding sites in the target nucleic acid molecule. The probe-binding sites of the target nucleic acid molecule are the regions of the target nucleic acid molecule which hybridise to the padlock probe, via its target-binding regions.

To allow ligation, the 3' and 5' ends which are to be ligated (the "ligatable" 3' and 5' ends) are hybridised to the target molecule or sequence, which acts as the ligation template. The binding of the padlock probe brings the ends into said juxtaposition. Where the complementary binding sites in the target molecule or sequence lie directly adjacent (or contiguous) to one another, the ends of the padlock probe will hybridise directly adjacent to each other (i.e. with no gap) and may be ligated to each other directly. Thus, in this case the ligatable ends of the probe are provided by the actual ends of the probe. In the case of a gap-fill padlock probe, the target binding regions at the ends of the padlock probe do not hybridise to adjacent binding sites, but rather to non-adjacent (non-contiguous) binding sites in the target molecule. In such an arrangement, the 5' ligatable end of the probe is provided by the actual 5' end of the probe. However, the ligatable 3' end of the probe is generated by extension of the hybridised 3' end of the probe, using the target sequence as extension template to fill the gap between the hybridised ends of the probe. The extension reaction brings the extended 3' end of the probe into juxtaposition for ligation. In this case, the ligatable 3' end of the probe is thus the extended 3' end of the probe.

Padlock probes may be provided in 2 or more parts that are ligated together. This may involve the provision of an additional ligation template, for example in the case of a 2-part probe, where each part comprises only one target-binding region, and the other end of each part hybridises to a common ligation template. In another embodiment, a 2-part padlock may take the form of a "connector" or "backbone" oligonucleotide with two target-binding regions at or near the 5' and 3' ends respectively, which hybridise to the target with a gap in between them, and a gap oligonucleotide which hybridises in the gap between the ends. Thus, in this embodiment, gap-filling between the hybridized ends of a padlock probe occurs by means of the gap oligonucleotide. The gap oligonucleotide may partially or fully fill the gap. In the case of an "extension gap-fill" padlock probe, the 3' end which is extended may be the hybridized 3' end of the gap oligonucleotide or the backbone oligonucleotide.

In a typical embodiment, however, the padlock probe is provided as a single circularisable oligonucleotide, whether as a gap-fill padlock, or not.

In particular embodiments the padlock probe does not have secondary structure, and more particularly does not comprise intramolecular double-stranded regions or stem-loop structures. However, dumbbell probes, which do have secondary structure, are a particular sub-type of padlock probe. The dumbbell probe comprises two stem-loop structures, joined stem to stem, wherein one of the "loops" is not closed, but is open with free 5' and 3' ends available for ligation to each other. This "open loop" functions as the target-binding domain of the probe. The closed loop functions simply as a spacer to join the end of the duplex (stem). In other words, it can be seen as a padlock probe with a region of duplex formed between complementary sequences (regions) of the padlock. The region of duplex functions as a signalling domain to which an intercalating agent can bind. Thus the "open loop" of a dumbbell probe may comprise the target-binding regions of complementarity.

The padlock probe may further comprise one or more other regions or sequences which may be useful in the method. This may include in particular a detection sequence which allows the probe to be detected, or identified. A detection sequence may for example be an identificatory sequence, such as a barcode sequence which may be detected to detect the padlock probe (more particularly, the complement of the detection sequence in the RCP or other amplification product may be detected). This may be detected for example by sequencing, or some other sequence-based detection method. Conveniently, the detection sequence, or more particularly its complement, may be detected by means of a detection oligonucleotide, which hybridises to the complement of the detection sequence in the RCP. The detection oligonucleotide is thus a detection probe, which is detected in order to detect the amplification product, e.g. RCP, and thereby the padlock probe which was used to generate it, thereby detecting the target nucleic acid molecule. The detection oligonucleotide may be provided with a detection moiety as discussed further below. A complementary copy of the detection sequence in the padlock probe is generated in each monomer repeat in the RCP. This complementary copy in the RCP is capable of hybridising to the detection oligonucleotide and may thus itself be complementary to the detection oligonucleotide. It can thus be seen that the detection sequence in the padlock probe may correspond to the sequence of the detection oligonucleotide which is used to hybridise to the RCP.

The blocking oligonucleotide is a linear oligonucleotide which hybridises to the target binding regions of the padlock probe. That is to say, the blocking oligonucleotide hybridises to both the 3' and 5' target binding regions of the padlock probe. When hybridised to the target binding regions of the padlock probe, the blocking oligonucleotide holds the 5' and 3' ends of the padlock probe in an arrangement whereby they are unable to be ligated to each other. For example, where the ends of the padlock probe are designed to hybridise to the target sequence directly adjacent to each other, they can be hybridised to the blocking oligonucleotide with a gap in between them, preventing their direct ligation (Fig. 1A). In another embodiment, the ends of the padlock probe, when hybridised to the blocking oligonucleotide, are arranged such that the 3' and 5' ends do not face each other, but rather extend away from each other (**Fig. 1B**). Thus, in the complex, the padlock hybridisation sequences (the sequences which hybridise to the target binding regions of the padlock probe) are hybridised to the target-binding regions of the padlock probe, and the target-binding regions of the padlock probe are arranged such that the 3' and 5' ends of the padlock probe cannot be hybridised to each other.

The padlock hybridisation sequences may be located at the 3' and 5' ends of the blocking oligonucleotide (as shown in Fig. 1) or may alternatively be located away from the ends of the blocking oligonucleotide, i.e. the blocking oligonucleotide may comprise one or two overhangs which extend beyond the padlock hybridisation sites. The padlock hybridisation site which binds the 5' end of the padlock may be located 3' to the padlock hybridisation site which binds the 3' end of the padlock, as shown in Fig. 1B. Alternatively, the padlock hybridisation site which binds the 5' end of the padlock may be located 5' to the padlock hybridisation site which binds the 3' end of the padlock, as shown in Fig. 1A. The padlock hybridisation sites may be any length which enables hybridisation of the padlock probe to the blocking oligonucleotide, e.g. at least 6, 7 or 8 nucleotides. The precise length is not critical as this may depend on the composition of the oligonucleotide, for example the GC content. In an embodiment, the padlock hybridisation sites are at least 8 nucleotides long. In particular embodiments, the padlock hybridisation sites may be 8-10, 8-12 or 8-14, 8-15 or 8-20 nucleotides long.

Generally, the padlock hybridisation sites of the blocking oligonucleotide are separated by a gap, i.e. a linker sequence (also referred to as a gap sequence herein). Thus, in the complex applied to the sample the target-binding regions of the padlock probe are separated by a gap. The gap is at least 1 nucleotide in length, preferably more than 1 nucleotide in length. In an embodiment, the gap is at least 6, 8 or 10 nucleotides long. Such a longer gap sequence may serve as a toehold region. A toehold region, as described further below, is a region accessible for another oligonucleotide or nucleic acid molecule to bind. However, the toehold region can also lie at either end of the blocking oligonucleotide. Thus, the blocking oligonucleotide may, in an embodiment, comprise a toehold region at an end of the oligonucleotide, outside a padlock hybridisation site. In such an embodiment, the gap region can be short as 1 nucleotide.

Accordingly, preferably the blocking oligonucleotide comprises at least one toehold region. A toehold region is a region of the blocking oligonucleotide which does not hybridise to the padlock probe (i.e. is located outside the padlock hybridisation sites) and is long enough to allow a second oligonucleotide (e.g. the target oligonucleotide) to invade the complex and displace the blocking oligonucleotide from the padlock probe. The toehold region is commonly located between the padlock hybridisation sites (i.e. it is commonly formed by the gap between the padlock hybridisation sites) but may instead be located at the 3' or 5' end of the blocking oligonucleotide, if the blocking oligonucleotide comprises an overhang at at least one end. The blocking oligonucleotide generally contains a toehold region to allow efficient displacement of the blocking oligonucleotide from the complex by a competing oligonucleotide. However, this is unnecessary in some embodiments in which the blocking oligonucleotide is degraded or competed away by ligation template, rather than displaced from the complex.

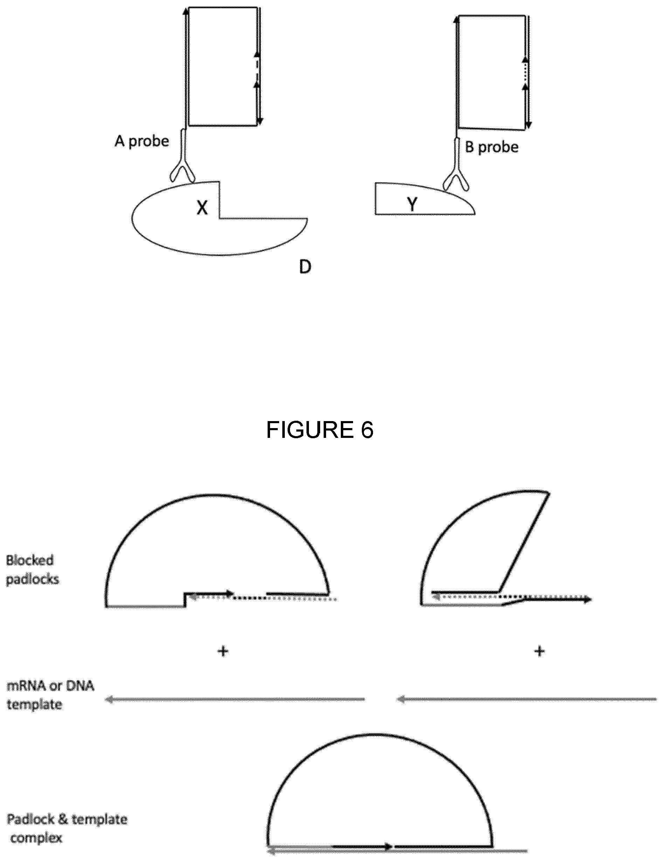

In an alternative to the inclusion of one or more toehold regions in the blocking oligonucleotide to allow its displacement, an equivalent role may be performed by an anchor sequence located within the padlock probe. An anchor sequence as defined herein is a continuation of a target-binding sequence within a padlock probe, which extends beyond the region that is complementary to the blocking oligonucleotide. That is to say, an anchor sequence is a part of a target-binding region which is upstream or downstream of the section bound by the blocking oligonucleotide. As part of a target-binding region, an anchor sequence is complementary to the target sequence (in the target nucleic acid molecule). Therefore, the anchor sequence is capable of hybridising to the target sequence when the blocking oligonucleotide is bound to the padlock probe, anchoring the padlock probe to the target sequence. Binding of the anchor sequence to the target sequence causes the target sequence to outcompete and displace the blocking oligonucleotide from the padlock probe. Padlock probes comprising anchor sequences are shown in Fig. 6.

As noted above, both the padlock probe and the blocking oligonucleotide are nucleic acid molecules. Generally, both the padlock probe and blocking oligonucleotide are DNA molecules.

Thus, the sample analysed according to the method herein is contacted with a complex comprising a padlock probe hybridised to a blocking oligonucleotide.

After contacting of the sample, removal of the blocking oligonucleotide from the complex is caused, such that the target-binding regions of the padlock probe hybridise to the probe-binding sites of the target nucleic acid molecule. This hybridisation of the target-binding regions of the padlock probe to the target nucleic acid molecule results in an arrangement of the target-binding regions which allows direct or indirect ligation of the target-binding regions to each other, as discussed above. Direct ligation of the target-binding regions is enabled by hybridisation of the 3' and 5' ends of the padlock probe directly adjacent to each other, such that the 3' end of the padlock probe can be ligated directly to the 5' end. As detailed above, indirect ligation of the target-binding regions is enabled by hybridisation of the target-binding regions to the target nucleic acid with a gap between them, i.e. by using a gap-fill padlock probe.

Removal of the blocking oligonucleotide from the complex can be performed by any suitable mechanism. In a particular and preferred embodiment, removal of the blocking oligonucleotide from the complex is performed passively, whereby the blocking oligonucleotide is displaced from the complex by the target nucleic acid molecule.

In this embodiment, the hybridisation of the probe-binding sites of the target nucleic acid to the target-binding sites of the padlock probe is favoured, due to high local concentration, over hybridisation of the padlock probe to the padlock hybridisation sites of the blocking probe. Therefore, when the complex is brought into close proximity with the target nucleic acid molecule, the target nucleic acid molecule out-competes and displaces the blocking oligonucleotide from the complex.

A higher affinity for the padlock probe of the probe-binding sites of the target nucleic acid molecule can be readily and straightforwardly achieved. For example, the probe-binding sites of the target nucleic acid molecule may be longer than the padlock hybridisation sites of the blocking oligonucleotide, and thus form longer duplexes with the padlock probe target-binding sites. By hybridising to the padlock probe over a longer stretch of nucleic acid, and forming longer duplexes, the probe-binding sites of the target nucleic acid molecule will have higher affinity for the padlock probe than the blocking oligonucleotide.

Alternatively, the probe-binding sites of the target nucleic acid molecule may simply display higher complementarity for the target-binding sites of the padlock probe (i.e. comprise fewer mismatches) than the padlock hybridisation sites of the blocking oligonucleotide. As is well known in the art, one nucleotide sequence will hybridise to another if the two sequences are complementary (that is to say if one of the sequences is the reverse complement of the other). However, it is not necessary for two sequences to be perfect complements of each other (i.e. 100 % complementary) in order to hybridise to each other. It is possible and common for sequences comprising a number of mismatches relative to each other nonetheless to hybridise to each other. By "mismatch" is meant, as standard in the art, a nucleotide which does not base pair with the nucleotide in the corresponding position in the other strand.

The higher the number of mismatches in a pair of nucleotide sequences, the lower the level of complementarity and the weaker the hybridisation. Thus, in the present instance, if the target nucleic acid displays a higher level of complementarity to the target-binding sequences than does the blocking oligonucleotide, it will displace the blocking oligonucleotide from the complex.

In a particular embodiment of the invention the blocking oligonucleotide comprises at least one mismatch to the target-binding sequences of the padlock probe, for instance the padlock hybridisation sequences of the blocking oligonucleotide may be at least 70, 75, 80, 85 or 90 % complementary to the target-binding sequences of the padlock probe (but less than 100 % complementary). In this embodiment the probe-binding sites of the target nucleic acid molecule comprise fewer mismatches to the target-binding sequences of the padlock probe than do the padlock hybridisation sequences of the blocking oligonucleotide. For instance, the probe-binding sites of the target nucleic acid may be at least 75, 80, 85, 90 or 95 % complementary to the target-binding sites of the padlock probe. In a particular embodiment the probe-binding sites of the target nucleic acid are 100 % complementary to the target-binding sites of the padlock probe.

Alternatively, competitive displacement of the blocking oligonucleotide from the complex by the target nucleic acid may rely on higher local concentration of the target nucleic acid than the blocking oligonucleotide, rather than stronger hybridisation to the padlock probe. In this embodiment, hybridisation of the padlock probe to the blocking oligonucleotide may be no weaker than to the target nucleic acid. For instance, the padlock hybridisation sites of the blocking oligonucleotide may have the same sequences as the probe-binding sites of the target nucleic acid. Indeed, where the blocking oligonucleotide comprises a gap between the hybridised ends of the padlock probe, and no gap is present when the padlock probe is hybridised to the target nucleic acid, the padlock hybridisation sites of the blocking oligonucleotide may be slightly longer than the probe-binding sites of the target nucleic acid, for instance 1-5 nucleotides longer. The longer hybridisation sites compensate for the loss of base stacking resulting from the gap between the padlock probe target-binding sites when they are hybridised to the blocking oligonucleotide.

In the context of proximity assays, whereby a padlock probe is brought into proximity to its target nucleic acid via binding of proximity probes to their targets (as discussed further below), the initial overall concentrations of padlock probe and target nucleic acid are relatively low, and the local concentration of blocking oligonucleotide in the context of the padlock probe is relatively high. Therefore, the complex between blocking oligonucleotide and padlock probe remains intact. When the padlock probe is brought into proximity to its target nucleic acid via binding of proximity probes to their targets, the local concentration of the target nucleic acid molecule to the padlock probe increases and becomes higher than the local concentration of the blocking oligonucleotide. Due to the higher local concentration of the target nucleic acid relative to the blocking oligonucleotide, the target nucleic acid is able to outcompete and displace the blocking oligonucleotide from the complex.

In another embodiment, removal of the blocking oligonucleotide from the complex is performed actively. This may be achieved using a key oligonucleotide, which is complementary to the blocking oligonucleotide and thus can hybridise to the blocking oligonucleotide, displacing it from the complex with the padlock probe. To enable displacement of the blocking oligonucleotide with the key oligonucleotide, the blocking oligonucleotide hybridises more strongly to the key oligonucleotide than it does to the padlock probe.

As an oligonucleotide capable of hybridising to the blocking oligonucleotide, the key oligonucleotide comprises sequences which are similar to the target-binding sequences of the padlock probe, and therefore may be capable of binding to the target nucleic acid. In order to avoid disrupting the binding of the padlock probe to the target nucleic acid, the key oligonucleotide must hybridise to the target nucleic acid less strongly than does the padlock probe. Preferably the key oligonucleotide does not hybridise to the target nucleic acid at all.

A key oligonucleotide as referred to herein is thus an oligonucleotide, generally linear, generally DNA, which is complementary to the blocking oligonucleotide. In this embodiment, the blocking oligonucleotide requires one or more toehold regions, forming one or more overhangs at the end of the blocking oligonucleotide (beyond the padlock hybridisation sites) and/or in the gap between the padlock hybridisation sites. In order to strongly hybridise to the blocking oligonucleotide, the key oligonucleotide displays a high level of complementarity to the toehold regions of the blocking oligonucleotide, e.g. the key oligonucleotide may be at least 90 or 95 % complementary to the toehold regions. The key oligonucleotide may indeed be 100 % complementary to the toehold regions. The key oligonucleotide however displays less than 100 %, preferably substantially less than 100 % complementarity, to the padlock hybridisation sites of the blocking oligonucleotide. The precise level of complementarity is not critical and may vary depending on the nature of the reagents involved (e.g. their length and/or nucleotide composition, etc.) A low, or reduced, level of complementarity may be achieved by the inclusion of one or multiple base mismatches and/or truncations in the regions of the key oligonucleotide corresponding to the padlock hybridisation sites of the blocking oligonucleotide.

The high level of complementarity of the key oligonucleotide to the toehold regions of the blocking oligonucleotide, coupled with the relatively low (or simply, lower) level of complementarity of the key oligonucleotide to the padlock hybridisation sites of the blocking oligonucleotide, enable the key oligonucleotide to bind the blocking oligonucleotide and displace it from the complex, while not hybridising, or only weakly hybridising, to the target nucleic acid. Use of a key oligonucleotide to disrupt the complex is shown schematically in Fig. 2.

Another active means of removing the blocking oligonucleotide from the complex is enzymatic digestion of the blocking oligonucleotide. In an embodiment, enzymatic digestion of the blocking oligonucleotide is enabled by the inclusion of one or more uridine residues in the blocking oligonucleotide. When the blocking oligonucleotide comprises one or more uridine residues, it is preferred that it includes multiple uridine residues, e.g. at least 2, 3, 4 or 5. Degradation of the blocking oligonucleotide can then be achieved by contacting the complex with uracil-DNA glycosylase (UDG) and an endonuclease enzyme.

As is well known in the art, uracil-DNA glycosylase excises uracil residues from within a DNA strand. The endonuclease is then able to work from the locations of the excised uracil bases to degrade the blocking oligonucleotide. Any suitable endonuclease may be used for this purpose, e.g. endonuclease IV.

Enzymatic degradation of the blocking oligonucleotide can alternatively be achieved using other endonucleases, including restriction enzymes and nickases etc. The skilled person is readily able to design cleavage sites or regions for degradation by cleavage. Restriction sites may for example be included in hairpin regions etc., and indeed endonucleases such as endonuclease IV can be used.

Alternatively, to enzymatic degradation of the blocking oligonucleotide, the blocking oligonucleotide may be degraded by photo-cleavage. Photo-cleavage is the cleavage of a molecule by exposure to light, i.e. the blocking oligonucleotide may be degraded by exposure to light. Photo-cleavage may also be referred to as photolysis, and takes places when absorption of a photon causes cleavage of a compound.

In general, photo-cleavage is achieved by the inclusion of one or more photo-cleavable linkers in the blocking oligonucleotide. Photo-cleavable linkers are chemical moieties which undergo cleavage upon exposure to light, also known as photolabile groups. Photo-cleavable linkers are well-known in the art, and generally are reactive to a light of a specific wavelength which causes cleavage of the group, commonly ultraviolet light. One or more photo-cleavable linkers may be included between nucleotides in the blocking oligonucleotide. Preferably, if the blocking oligonucleotide is to be cleaved by photolysis, it comprises at least 2, e.g. 3, 4 or 5 photo-cleavable linkers. Exemplary photo-cleavable are reviewed in LeValley et al., J. Am. Chem. Soc. 2020, 142, 10, 4671-4679.

When the complex is to be passively dissociated, this takes place simply by contacting the sample comprising the target nucleic acid molecule with the complex of the blocking oligonucleotide and padlock probe. When the complex is to be actively dissociated the dissociation is performed following the contacting of the sample with the complex. Thus, in this case, the sample is contacted with the complex, and the sample/complex mixture then contacted with e.g. the key oligonucleotide or the enzyme(s) to degrade the blocking oligonucleotide, e.g. one or more restriction enzymes or UDG plus an endonuclease, or alternatively if a photo-cleavable blocking oligonucleotide is used, light of a suitable wavelength to cleave the photo-cleavable linker(s) is applied to the sample/complex mixture.

The reaction mixture may be incubated in conditions appropriate to facilitate or enable padlock probe binding (the so-called "annealing" step). Conditions for this step are known in the art, and are within the routine skill of the skilled practitioner in the art to select or design. For example, an annealing temperature of room temperature, or in the range of 20-40°C may be used, e.g. 25-40°C, or 25-37°C. In one embodiment a higher temperature, e.g. 50-65°C may be used, e.g. 53-60°C, or 55-60°C. If an elevated annealing temperature is selected, the annealing temperature may be reduced for the extension step if a gap-fill padlock is used. Again, the appropriate conditions can be selected according to what is known in the art, and the particular reagents, e.g. enzymes used. For example, after the initial annealing step, the temperature may be reduced to 28-40°C, e.g. 28-35, 30-35, 28-33, 30-33 or 30-32°C, etc.

Once the complex of the blocking oligonucleotide and padlock probe has been dissociated and the padlock probe bound to its target nucleic acid molecule, the reaction mixture may be washed. The inclusion of such a step is, however, not necessary or critical. In one embodiment a washing step may optionally be included if the blocking oligonucleotide was actively removed from the complex, such that the padlock probe was all released and free padlock probe is thus present in the reaction mixture. Generally speaking, though, a washing step is not necessary, since the padlock probe may be designed to have a better hybridisation strength to its target (i.e. to its ligation template) than to the blocking oligonucleotide. Further, the key oligonucleotide may be designed to minimise hybridisation strength to the target nucleic acid molecule.

The target-binding regions of the padlock probe bound to its target nucleic acid molecule are then ligated to each other, directly or indirectly as appropriate for the probe. If a gap-fill probe is used, an extension step is first performed, for which a DNA polymerase and dNTPs are added to the reaction mixture. Extension of the 3' end of the padlock probe is performed using the target nucleic acid gap sequence as template and using a DNA polymerase lacking strand-displacement activity or 5'-3' exonuclease activity, such as T4 or T7 DNA polymerase.

For ligation of the padlock probes any convenient ligase may be employed, and representative ligases of interest include, but are not limited to, temperature sensitive ligases such as SplintR ligase (also known as PBCV-1 DNA ligase or Chlorella virus DNA ligase) bacteriophage T4 DNA ligase, bacteriophage T7 ligase, and *E. coli* ligase, and thermostable ligases such as Taq ligase, Tth ligase, Ampligase^{®}, Pfu ligase and 9°N^{™} DNA Ligase.

Suitable conditions for ligation are known in the art, and any reagents that are necessary and/or desirable may be combined with the reaction mixture and maintained under conditions sufficient for ligation. It will be evident that the ligation conditions may depend on the ligase enzyme used in the methods of the invention. Thus, for example, Ampligase may be used, and the temperature may be increased for the ligation step. Alternatively, SplintR ligase may be used at room temperature.

Where temperature change or temperature control steps are required, the method may be performed in a thermal cycling instrument. This permits a ready control of the temperature changes. However, an advantage of the method is that extreme temperature changes are not necessary, and the method may for example be performed at room temperature, or at 20-37°C for example. Probe binding and ligation steps may for example be performed at room temperature.

The conditions for the probe binding and ligation reactions may be optimised by routine experimentation according to principles known in the art. Thus, temperature, buffers, time of incubation, ramping speed etc. may be adjusted to find the optimal conditions.

Ligation of the padlock probes circularises the padlock probes, thus yielding one or more circular products. The circularised padlock probe, or a part thereof, is then amplified using a standard technique known in the art.

In a preferred embodiment the entire circularised padlock probe is amplified using rolling circle amplification (RCA). RCA utilises a strand displacement polymerase enzyme, and requires a circular amplification template, which may be provided by a circularised padlock probe. Amplification of the circular template provides a concatenated RCA product (RCP), comprising multiple copies of a sequence complementary to that of the amplification template (thus an RCP from a padlock probe comprises repeating units corresponding to the padlock probe). Such a concatemer typically forms a ball or "blob", which may readily be visualised and detected, and thus RCA-based assays have been adopted for the detection of nucleic acids, and indeed, more generally, as reporter systems for the detection of any target analyte.

Before the RCA reaction there may be an optional washing step (again this can only be performed if the target nucleic acid molecule is immobilised). An RCA reaction is then typically initiated by adding one or more reagents for the RCA (termed "RCA reagents"). This will typically be the polymerase enzyme for RCA and nucleotides (specifically dNTPs), although optionally a primer for RCA may also be added. One or more of the RCA reagents may be added earlier, as long as all the RCA reagents are not present earlier. In an embodiment, the RCA reaction is initiated by adding at least a polymerase enzyme.

The primer for the RCA reaction may be added to the reaction mixture, or may be pre-hybridised to the padlock probe. The binding site for the RCA primer may be provided in a region of the padlock probe which is different to the target binding regions (i.e. in the backbone region of the padlock). In some cases the target nucleic acid molecule may serve as or provide the primer. The strand-displacing polymerase enzyme used for RCA is commonly Phi29 or a derivative thereof.

Alternatively, a part of the circularised padlock probe may be amplified by a standard DNA amplification technique such as PCR. Specifically, a section of the circularised padlock probe including the ligation site may be amplified, since such a product can only be generated from ligated, circularised padlock probe. To perform this amplification reaction, following ligation of the padlock probe primers, dNTPs and a polymerase enzyme are added to the reaction mixture and the amplification performed following a standard protocol.

The amplified padlock probe or part thereof (that is to say, the amplification product) is then detected. As set out above, the detection process may be a simply binary process, determining whether the amplification product is present or absent. Alternatively, the detection may be quantitative or semi-quantitative.

Any suitable detection method may be used to detect the amplification product, depending on the nature of the amplification product. Commonly, the padlock probe comprises a detection sequence, as described above, which as previously detailed can be detected in the context of a rolling circle amplification product, or indeed another amplification product, using a detection oligonucleotide. A detection sequence may be found, for example, in a portion of the backbone region of the padlock probe, that is the region between the target-binding regions. In a dumbbell probe it may be in the duplex region of the probe.

In particular, the padlock probe may contain a detection sequence by which it may be detected. A complement of the detection sequence will become incorporated into the RCP, and may be detected, for example by the binding to it of a detection probe, or by sequencing. The detection sequence may be specific to the padlock probe, and thus to the target sequence, or sequence variant it is desired to detect. Thus, in multiplex methods of the invention each padlock probe may have a different detection sequence. The detection sequence may be detected to detect or identify which padlock probe was amplified in the RCA, and hence which target sequence was present. Such a protocol may be applied, for instance, in the context of a method for detecting a target sequence variant, where each padlock is provided with a detection sequence specific to particular variant. The detection sequence may thus be seen as a marker or identification sequence. The term "detection sequence" as used herein includes both the detection sequence as it occurs in the padlock probe, and the complementary copy, e.g. as it appears in the RCP.

Accordingly, a detection sequence may be used to "tag" or mark different padlock probes so that they, or their ligation or amplification products, may readily be distinguished from one another. Additionally, such a sequence may be used to tag different samples etc. for example so that they may be pooled (i.e. a "sample" tag or marker). Thus, in a multiplex setting, different probes (i.e. probes for different target nucleic acid sequences or different variants) may be provided with different tag sequences (e.g. different marker or detection sequences) and/or they may be provided with the same tag sequence(s) e.g. for the introduction of a common or universal sequence. Such methods may be used in conjunction with particular detection methods, including the use of detection probes or sequencing methods such as sequencing by hybridisation, sequencing by ligation or other next generation sequencing chemistries, e.g. in the multiplexed detection of multiple target nucleic acids in a sample.

The detection oligonucleotide used in the method may carry a detectable label, also referred to as a detection moiety. The detection moiety is any moiety which can be detected, that is which can give rise, directly or indirectly, to a signal which can be detected. The detection moiety may thus be viewed as any detectable label, which may be directly or indirectly signal-giving. For example, the detection moiety may be spectroscopically or microscopically detectable, e.g. it may be a fluorescent or colorimetric label, a particle, e.g. a bead, or an enzymatic label. Any of the labels used in immunohistochemical techniques may be used. Hybridisation of the detection oligonucleotides to their multiple binding sites in an RCP (i.e. the repeating detection sequence), concentrates them in the RCP, allowing it to be detected with high sensitivity.

The detection oligonucleotide need not, however, be directly labelled. For example, the detection oligonucleotide may be an unlabelled probe which functions as a sandwich probe. The concept of sandwich probes is well known in the art and may be applied according to any convenient protocol. The sandwich probes can bind to the RCP (or other amplicon) but are not directly labelled themselves; instead, they comprise a sequence to which labelled secondary oligonucleotides can bind, thus forming a "sandwich" between the RCP and the labelled secondary oligonucleotide. Alternatively, the RCP may be detected indirectly, e.g. the product may be amplified by PCR and the amplification products may be detected.

The detection oligonucleotide or any secondary labelling probe may be labelled with a directly or indirectly detectable label. A directly detectable label is one that can be directly detected without the use of additional reagents, while an indirectly detectable label is one that is detectable by employing one or more additional reagents, e.g., where the label is a member of a signal producing system made up of two or more components. In many embodiments, the label is a directly detectable label, where directly detectable labels of interest include, but are not limited to: fluorescent labels, coloured labels, radioisotopic labels, chemiluminescent labels, and the like. In many embodiments, the label is a fluorescent label, where the labelling reagent employed in such embodiments is a fluorescently tagged nucleotide(s), e.g. fluorescently tagged CTP (such as Cy3-CTP, Cy5-CTP) etc. Fluorescent moieties which may be used to tag nucleotides for producing labelled probe nucleic acids (i.e. detection probes) include, but are not limited to: fluorescein, the cyanine dyes, such as Cy3, Cy5, Alexa 555, Bodipy 630/650, and the like. Other labels, such as those described above, may also be employed as are known in the art.

Conveniently, the detection moiety may be a coloured bead. Coloured beads may readily be visualised. Such beads, e.g. coloured polystyrene beads, are widely available.

Although various detection modalities may be employed, conveniently RCPs may be detected by visualisation, including by microscopy, or flow cytometry. In both cases directly or indirectly labelled detection oligonucleotides may be used, for example with fluorescent or coloured labels which may readily be detected. In this regard the label may include a bead or other detectable particle. In a microscopy-based method, RCPs may be detected by imaging.

In an alternative embodiment, rather than the detection sequence being included in the padlock probe backbone, the detection sequence is formed by ligation of the padlock probe, particularly by extension and ligation of the probe in the context of a gap-fill probe. In particular, when performing a gap-filling step in the method, the gap sequence (in the blocking oligonucleotide and/or the target nucleic acid molecule) may act as a barcode sequence. In this manner, incorporation of a barcode sequence into a padlock probe by gap-filling enables the distinction of otherwise identical padlock probes based on the sequence to which they are bound.

For instance, the padlock probe may be hybridised to the blocking oligonucleotide in a manner as shown in **Fig. 1A****,** whereby a gap is located between the target-binding regions but, the padlock probe can be circularised by gap-filling and ligation. This gap-filling and ligation reaction results in incorporation of a blocking oligonucleotide-specific barcode sequence into the padlock probe. The padlock probe may hybridise to the target nucleic acid molecule in a manner which allows direct ligation of the target-binding regions (i.e. without a gap between them), or may hybridise to the target nucleic acid molecule with a gap between the target-binding regions, wherein the gap sequence is different to the gap sequence present in the blocking oligonucleotide. Thus, padlock probe hybridised to the target nucleic acid molecule can be distinguished from padlock probe which remains hybridised to the blocking oligonucleotide, either by the direct ligation of its target-binding regions or by the incorporation of a different, target-specific barcode sequence.

In this embodiment, in which the detection sequence is present only in a circularised padlock probe, the amplification step may entail an RCA reaction as set out above, or amplification of the detection sequence alone, e.g. by PCR. When amplified by RCA, the detection sequence is generally detected using a detection oligonucleotide, as discussed above. A detection oligonucleotide may also be used to detect an amplification product generated by PCR. For instance, qPCR using "TaqMan" probes may be performed. In this instance, a probe complementary to each different detection sequence is used with each different probe being conjugated to a different, distinguishable fluorophore. Each detection sequence (and thus each different amplification product) can thus be individually detected and quantified.

Indeed, qPCR may be used for detection of circularised padlock probes using either the circularised padlock probe directly as the PCR template (as mentioned above), or following RCA using the RCP as template.

Alternatively, as noted above the PCR product may be detected by sequencing. In this case, a form of high throughput DNA sequencing is preferably used, particularly when the method is performed in multiplex. Sequencing by synthesis is the preferred DNA sequencing method. Examples of sequencing by synthesis techniques include pyrosequencing, reversible dye terminator sequencing and ion torrent sequencing, any of which may be utilised in the present method. Preferably the detection sequences are sequenced using massively parallel DNA sequencing. Massively parallel DNA sequencing may in particular be applied to sequencing by synthesis (e.g. reversible dye terminator sequencing, pyrosequencing or ion torrent sequencing, as mentioned above). Massively parallel DNA sequencing using the reversible dye terminator method is a preferred sequencing method. Massively parallel DNA sequencing using the reversible dye terminator method may be performed, for instance, using an Illumina^{®} NovaSeq^{™} system.

As is known in the art, massively parallel DNA sequencing is a technique in which multiple (e.g. thousands or millions or more) DNA strands are sequenced in parallel, i.e. at the same time. Massively parallel DNA sequencing requires target DNA molecules to be immobilised to a solid surface, e.g. to the surface of a flow cell or to a bead. Each immobilised DNA molecule is then individually sequenced. Generally, massively parallel DNA sequencing employing reversible dye terminator sequencing utilises a flow cell as the immobilisation surface, and massively parallel DNA sequencing employing pyrosequencing or ion torrent sequencing utilises a bead as the immobilisation surface.

As is known to the skilled person, immobilisation of DNA molecules to a surface in the context of massively parallel sequencing is generally achieved by the attachment of one or more sequencing adapters to the ends of the molecules. Thus, in the method herein, when the detection sequence is amplified by PCR, the PCR primers may comprise adapters for sequencing (sequencing adapters) for addition to the detection sequences, to enable sequencing of the products. Commonly, sequencing adapters are nucleic acid molecules (in particular DNA molecules). In this instance, short oligonucleotides complementary to the adapter sequences are conjugated to the immobilisation surface (e.g. the surface of the bead or flow cell) to enable annealing of the target DNA molecules to the surface, via the adapter sequences. Alternatively, any other pair of binding partners may be used to conjugate the target DNA molecule to the immobilisation surface, e.g. biotin and avidin/streptavidin. In this case biotin may be used as the sequencing adapter, and avidin or streptavidin conjugated to the immobilisation surface to bind the biotin sequencing adapter, or vice versa.

Sequencing adapters may thus be short oligonucleotides (preferably DNA), generally 10-30 nucleotides long (e.g. 15-25 or 20-25 nucleotides long). As detailed above, the purpose of a sequencing adapter is to enable annealing of the target DNA molecules to an immobilisation surface, and accordingly the nucleotide sequence of a nucleic acid adaptor is determined by the sequence of its binding partner conjugated to the immobilisation surface. Aside from this, there is no particular constraint on the nucleotide sequence of a nucleic acid sequencing adaptor.

In general, where amplification of the nucleic acid products is performed by RCA, the RCP is conveniently detected using a specific, labelled detection oligonucleotide. Where amplification is performed by PCR, detection is generally conveniently performed by qPCR or sequencing. However, the detection method is not critical and may be varied according to choice.

According to the methods herein, detection of circularised padlock probe bound to its target nucleic acid molecule is a proxy for detection of the target nucleic acid molecule. Thus, detection of circularised padlock probe bound to its target nucleic acid molecule indicates the presence of the target nucleic acid molecule in the sample.

As noted above, detection of the target nucleic acid molecule may be quantitative or semi-quantitative. The quantification may be relative, i.e. the relative amounts of two or more target nucleic acid molecules may be compared. Relative quantification can be accomplished by comparison of detected levels or amounts between two or more different target molecules, to provide a relative quantification of each of the two or more different nucleic acid molecules or sequences, i.e., relative to each other. Thus, ratios of target molecules present in a sample may be determined.

Alternatively, the quantification may be absolute, whereby the concentrations of one or more target nucleic acid molecules in the sample are calculated. Absolute quantification generally requires generation of a standard curve using known concentrations of the target molecules against which the detection values can be compared.

In a particular embodiment, the circularised padlock probe is amplified by RCA. The RCP is detected and the amount of the target quantified by RCA signal counting according to techniques and principles well known in the art. It is known for example to detect RCPs by means of labelled detection probes specific for RCP, but means of which they may be visualised, e.g. imaged, and counted. Image analysis software is available for such use.

Alternatively, quantification of a target detected by qPCR is routine in the art, including absolute quantification qPCR using a standard curve. The levels of one or more target molecules can also be quantified during sequencing, if sequencing is used for detection of the amplification products. The level of amplification product generated should be proportionate to the amount of target present in the sample. Use of a common primer binding site for amplification helps to ensure that the levels of different amplification products are comparable and proportionate to their concentrations. The levels of each amplification product sequenced are detected during sequencing allowing their relative concentrations to be calculated. Again, generation of a standard curve using known concentrations of each target molecule, against which the experimental values can be compared, allows absolute quantification of the target molecule concentrations.

The term "hybridisation" or "hybridises" as used herein refers to the formation of a duplex between nucleotide sequences which are sufficiently complementary to form duplexes via Watson-Crick base pairing, or any analogous base-pair interactions. Hence, a region of complementarity in a molecule or probe or sequence refers to a portion of that molecule or probe or sequence that is capable of forming a duplex. As mentioned above, hybridisation does not require 100 % complementarity between the sequences, and hence regions of complementarity to one another do not require the sequences to be fully complementary, although this is not excluded. Thus, the regions of complementarity may contain one or more mismatches. Accordingly, "complementary", as used herein, means "functionally complementary", i.e. a level of complementarity sufficient to mediate a productive hybridisation, which encompasses degrees of complementarity less than 100 %. The degree of mismatch tolerated can be controlled by suitable adjustment of the hybridisation conditions. Those skilled in the art of nucleic acid technology can determine duplex stability empirically considering a number of variables including, for example, the length and base pair composition of the respective molecules or probe oligonucleotides, ionic strength, and incidence of mismatched base pairs, following the guidance provided by the art. Thus, the design of appropriate probes, and binding regions thereof, and the conditions under which they hybridise to their respective targets is well within the routine skill of the person skilled in the art.

A region of complementarity, such as for example to a target sequence in the binding region of a padlock probe, or between a detection sequence and a detection oligonucleotide, or a RCA primer to the circularised padlock probe etc., may be at least 6 nucleotides long, to ensure specificity of binding, or more particularly at least 7, 8, 9 or 10 nucleotides long. The upper limit of length of the region is not critical, but may for example be up to 50, 40, 35, 30, 25, 20 or 15 nucleotides. A complementary region may thus have a length in a range between any one of the lower length limits and upper length limits set out above. In the case of a padlock probe, the length of an individual target-binding region may be in the lower ranges, so that the total length of the two binding regions when hybridised to their target is within the upper ranges. For example, an individual target binding region may be 8-15, e.g. 10-12 nucleotides, so that the total hybridised length is 16-30 nucleotides long, e.g. 20-24. It may be desirable, within the constraints of conformation of the probes, and spacing of the domains, and desired or favoured hybridisations, to minimise the total length of a padlock probe, particularly when it will be amplified by RCA, to minimise the size of the circle which is subjected to RCA, and hence to minimise the lengths of the complementary regions where possible.

As mentioned above and set out below, the target nucleic acid molecule may be an analyte, or it may be a nucleic acid molecule which is used or generated as a proxy, or a reporter or indicator, for the analyte to be detected.

Thus, in one embodiment, the target nucleic acid recognised by the padlock probe is itself an analyte, i.e. the subject of the method which it is desired to detect. In this embodiment the analyte is a particular nucleic acid or nucleotide sequence in a sample. For instance, it may be a nucleic acid belonging to a pathogen (e.g. a virus or bacterium), which the method of the invention is used to detect, or may be derived from such a nucleic acid (for instance the target nucleic acid may be cDNA produced by reverse transcription of the genome of an RNA virus).

In another embodiment, the target nucleic acid recognised by the padlock probe is a reporter for a target molecule. As used herein, a reporter for a target molecule may be generated from (i.e. as a result of the presence of) a target molecule, or may be a proxy for the presence of a target molecule, as discussed above. Essentially, a reporter nucleic acid 'reports' the presence of a target analyte in the sample.

In this embodiment, the target analyte may be any analyte it is desired to detect. For instance, the analyte may be a nucleic acid, a protein (which term includes peptides and polypeptides), or any other chemical or biological molecule or moiety, including for example carbohydrates, e.g. such as may occur as glycosyl groups on proteins. The target analyte may thus be a modified protein, for example with a post-translational modification (PTM) which is detected in an assay for the analyte.

Thus, the target nucleic acid may be merely a reporter nucleic acid indicating the presence of an analyte of interest, in particular a protein.

In a particular embodiment the target nucleic acid molecule is a reporter nucleic acid comprised within a detection probe for an analyte. The detection probe for the analyte is any probe which is used in an assay to directly or indirectly bind and thereby detect the analyte of interest. Thus, the probe may be a primary probe, which directly binds to the analyte of interest, or a secondary probe, which binds to a probe which is in turn bound to the analyte of interest.

In a preferred embodiment the target nucleic acid molecule is comprised within a proximity probe, i.e. a probe which forms part of a set of proximity probes used together to detect a target analyte in a proximity assay, typically a proximity probe pair, and is suitable for use in a proximity ligation or proximity extension assay. Such an assay may be used for the detection of an analyte (as described in e.g. WO 01/61037), or of an interaction between two analytes, or a modification (e.g. PTM), where both the underlying analyte (e.g. protein) and the modifying group are detected, as further described below. Proximity assays may be performed in homogenously (i.e. in solution) or in a solid phase formats, and the present methods may be carried out in the context of any format of proximity assay which uses padlock probes, including both in-solution and solid phase formats.

As is well known in the art, a proximity probe comprises a binding domain and a nucleic acid domain. The nucleic acid domain may be single-stranded or partially double stranded. The binding domain of a proximity probe specifically binds the target molecule it is designed to detect. In this respect, the binding domain may bind to a target analyte directly or indirectly. In other words, the proximity probe may be a primary reagent, or a secondary reagent bound to an intermediate reagent (e.g. to a primary probe which binds directly to the analyte). The nature of a binding domain is dependent on the type of target molecule it is designed to detect. When the target molecule is a protein, the binding domain is generally an antibody or antigen-binding fragment or derivative thereof which is specific for the protein of interest. Examples of suitable antibody fragments and derivatives include Fab, Fab', F(ab')₂ and scFv molecules.

A Fab fragment consists of the antigen-binding domain of an antibody. An individual antibody may be seen to contain two Fab fragments, each consisting of a light chain and its conjoined N-terminal section of the heavy chain. Thus a Fab fragment contains an entire light chain and the V_{H} and C_{H}1 domains of the heavy chain to which it is bound. Fab fragments may be obtained by digesting an antibody with papain.

F(ab')₂ fragments consist of the two Fab fragments of an antibody, plus the hinge regions of the heavy domains, including the disulphide bonds linking the two heavy chains together. In other words, a F(ab')₂ fragment can be seen as two covalently joined Fab fragments. F(ab')₂ fragments may be obtained by digesting an antibody with pepsin. Reduction of F(ab')₂ fragments yields two Fab' fragments, which can be seen as Fab fragments containing an additional sulfhydryl group which can be useful for conjugation of the fragment to other molecules. ScFv molecules are synthetic constructs produced by fusing together the variable domains of the light and heavy chains of an antibody. Typically, this fusion is achieved recombinantly, by engineering the antibody gene to produce a fusion protein which comprises both the heavy and light chain variable domains.

As is well known in the art, when a pair of proximity probes is used to detect a single protein, the two antibodies in the pair (or antibody derivatives) bind the protein at different epitopes.

When the target molecule is a nucleic acid molecule, the binding domain is generally also a nucleic acid molecule. In this instance the binding domain nucleic acid molecule is at least partially single-stranded, and the single-stranded region of the binding domain is, or comprises a region which is, complementary to the target molecule, such that it specifically hybridises to it. When the binding domain is a nucleic acid molecule it may be a DNA or an RNA molecule.

The nucleic acid domain of the proximity probe in this context may of course be the target nucleic acid molecule recognised by the padlock probe. However, proximity assays may take various formats, and as noted above the padlock probe may be used in the assay in other ways. For example, a padlock probe may be used to detect the ligation product of a proximity ligation assay, e.g. wherein the two nucleic acid domains of a proximity probe are ligated together, or the extension product of a proximity extension assay, e.g. wherein the nucleic acid domains of a pair of proximity probes hybridise together and the one nucleic acid domain templates the extension of the other nucleic acid domain. More generally, therefore, the target nucleic acid molecule may be the product of an interaction between nucleic acid domains of proximity probes. Still further it may be an oligonucleotide reagent (including another padlock probe), which is used in conjunction with the proximity probes.

When the binding domain of the probe is an antibody the antibody is conjugated to the reporter nucleic acid. When the binding domain is a nucleic acid, the probe may be a single nucleic acid comprising the binding domain at one end and the reporter domain at the other, optionally joined by a linker nucleic acid sequence. Alternatively, the binding domain nucleic acid and the reporter nucleic acid domain may both be conjugated to a core group which is not a nucleic acid molecule.

Preferably, the proximity probe is for a protein, and thus comprises an antibody (or fragment or derivative thereof) conjugated to the target nucleic acid molecule.

Accordingly, in an embodiment, the method is for detecting a target analyte in a sample, wherein first and second proximity probes are used to detect the target analyte, each proximity probe being capable of binding directly or indirectly to the target analyte wherein the nucleic acid domain of one proximity probe is hybridised to a padlock probe, and the target binding regions of the padlock probe are capable of hybridising to probe-binding sites located in either the nucleic acid domain of the other proximity probe, or in an oligonucleotide (e.g. a padlock probe) hybridised to the nucleic acid domain of the other proximity probe.

In a particular embodiment the invention provides a method for detecting an interaction between two target molecules in a sample. The method is a variant of a proximity ligation assay, in which the sample is contacted with a pair of proximity probes, one probe specific for each of the two target molecules of interest. That is to say, the sample is contacted with a first proximity probe specific for the first target molecule and a second proximity probe specific for the second target molecule. As set out above, each proximity probe comprises a nucleic acid domain.

As mentioned, this embodiment of the invention is for detecting an interaction between two target molecules. The two target molecules in the sample are different molecules (i.e. different species of molecule). That is to say the method is not suitable for detecting homomeric interactions. The target molecules may be any type of biomolecules, and the two target molecules may be different types of biomolecule. Thus, a target molecule may be for example a protein or a nucleic acid molecule, e.g. a DNA molecule or an RNA molecule.

Most commonly, the target molecules are both proteins. The protein may be a natural (wild type) protein from a human or other organism, or it may be a synthetic protein (e.g. a fusion protein), a protein fragment or a modified protein or mutant protein. Thus, the method is most commonly for detecting the two proteins and the protein-protein interaction between them. In other embodiments however, the method may be used to detect e.g. protein-DNA or protein-RNA interactions.

The interaction between the two target molecules is an interaction in which the two target molecules come together. Generally, the interaction is a direct interaction, whereby the two molecules bind to one another, though it may be an indirect interaction whereby the two target molecules are joined via a connecting molecule. In any event, in order for the interaction to be detected the two target molecules must be in sufficiently close proximity that the proximity probes bound to them are able to interact with each other. The interaction may be a covalent interaction, but is generally a non-covalent interaction.

In at least one of the proximity probes used to detect the interaction between the target molecules, the nucleic acid domain is hybridised to a complex comprising a padlock probe and a blocking oligonucleotide. The nucleic acid domain is hybridised to the complex prior to being applied to the sample. The padlock probe is hybridised to the nucleic acid domain of the probe via a hybridisation sequence within the backbone of the padlock probe. One or both of the proximity probes may be hybridised to a padlock probe-blocking oligonucleotide complex in this manner, prior to being applied to the sample.

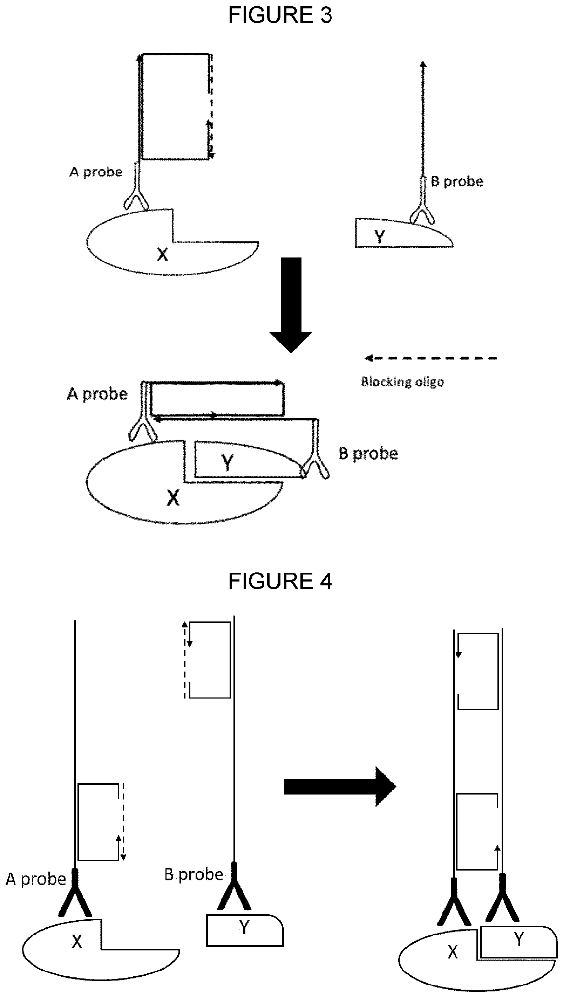

The target-binding regions of the padlock probe hybridise to probe-binding sites located in either the other nucleic acid domain (that is to say, the nucleic acid domain of the other proximity probe in the proximity probe pair) or, if the nucleic acid domains of both proximity probes in the pair are hybridised to padlock probe-blocking oligonucleotide complexes, then the target-binding regions of the two padlock probes may hybridise to each other. A schematic diagram of such an embodiment of the method is shown in **Figure 3****.** However, in a variant of the method, a key oligonucleotide may be used to remove the blocking oligonucleotide, to allow the padlock probe to hybridise to the other nucleic acid domain.

The two proximity probes, when applied to the sample, bind to their respective target molecules (assuming the target molecules are present in the sample). Interaction of the two target molecules brings the proximity probes into proximity of each other, allowing the target-binding regions of the padlock probe(s) to hybridise to their target nucleic acid molecule. Circularisation and ligation of the padlock probe(s) can then proceed. The circularised products are amplified and detected as described above.

This embodiment of the invention thus relies on the blocking oligonucleotide being displaced from the padlock probe when the two proximity probes come into proximity to each other. Therefore, in this embodiment the blocking oligonucleotide is competitively displaced by the target nucleic acid molecule. However, in a variant of the method, key oligonucleotides may be used to remove the blocking oligonucleotide(s) from the padlock probe(s).

In this way, circularisation of the padlock probe is only achieved when the target molecules interact and thus the ligation/amplification product of the padlock probe is indicative of an interaction between the two target molecules. That is to say, detection of the product of ligation and amplification of the padlock probe demonstrates that the two target molecules are interacting in the sample.

In this embodiment, in which an interaction between two target molecules in a sample is detected, the method preferably comprises:
(i) contacting the sample with a first proximity probe for detection of the first target molecule and a second proximity probe for detection of the second target molecule, wherein said proximity probes each comprise a binding domain and a nucleic acid domain, and said first and second proximity probe together form a proximity probe pair for detection of the interaction between the two target molecules;
   wherein the nucleic acid domain of the first proximity probe is hybridised to a first padlock probe-blocking oligonucleotide complex, and the nucleic acid domain of the second detection probe is hybridised to a second padlock probe-blocking oligonucleotide complex;
   wherein the target-binding regions of the first padlock probe are capable of hybridising to probe-binding sites in the nucleic acid domain of the second proximity probe or in the second padlock probe, and the target-binding regions of the second padlock probe are capable of hybridising to probe-binding sites in the nucleic acid domain of the first proximity probe or the first padlock probe;
   and wherein the blocking oligonucleotides are displaced from the padlock probes when the padlock probes are in close proximity to their target nucleic acid molecules, such that upon binding of the first and second proximity probes to interacting target molecules the padlock probes hybridise to their respective target nucleic acid molecules, while a padlock probe hybridised to a detection probe bound to a non-interacting target molecule remains in complex with its blocking oligonucleotide;
(ii) performing a gap-filling and ligation reaction, thereby generating circularised ligation products, wherein the ligation product from a padlock probe in complex with its blocking oligonucleotide comprises a barcode sequence from the blocking oligonucleotide, such that circularisation of the padlock probes generates distinct products indicating interacting and non-interacting target molecules;
(iii) detecting the circularisation products and their relative levels, thereby determining the proportion of each target molecule interacting with the other.

A schematic diagram demonstrating this embodiment of the method of the invention is presented in **Figure 4****.** The target binding regions of the padlock probes may hybridise to the padlock hybridisation sites in the nucleic acid domains of the other proximity probes with their 3' and 5' ends adjacent or non-adjacent (as shown).

In this embodiment, both padlock probes are circularised, using either their target nucleic acid molecule (the nucleic acid domain of the other proximity probe) or their blocking oligonucleotide as ligation template. The blocking oligonucleotide and, optionally, the target nucleic acid molecule, comprises a unique barcode sequence in the gap between the probe-binding sites to which the target-binding regions of the padlock probe binds, such that circularised padlock probes which remain bound to their blocking oligonucleotide can be distinguished from those which bind a target nucleic acid molecule. The first and second padlock probes can also be distinguished by their sequences. The target-binding regions of the two padlock probes are preferably different both to enable distinction between the two and to prevent either padlock probe binding to the target sequence of the other.

In this embodiment, circularised padlock probe which has remained bound to its blocking oligonucleotide (as indicated by the presence in it of the barcode from the blocking oligonucleotide) is indicative of free target molecule (or at least target molecule which is not bound to the other target molecule of interest). That is to say, when a padlock probe remains hybridised to its blocking oligonucleotide, this indicates target molecule bound by the associated proximity probe which is not interacting with its interaction partner of interest. Circularised padlock probe which bound its target nucleic acid sequence is indicative of interaction between the two target molecules. Quantification of the up to four amplification products of this method allows quantification of the total amount of each target molecule in the sample, and how much of each target molecule is free and how much is bound to the other target molecule.

In an alternative of this method, the first and second padlock/blocking oligonucleotide complexes may be used to detect the free (non-interacting) target molecules, and a third padlock probe/blocking oligonucleotide complex may be used to detect the interaction. This third padlock probe may be hybridised, or capable of hybridising, to the nucleic acid domain of one of the first and second proximity probes (at a different site to the first or second padlock), and its target-binding regions may be capable of hybridising to the nucleic acid domain of the other proximity probe (again at a different site). The construction of proximity probes for use in such a method may be facilitated by providing the nucleic acid domain of the proximity probe targeted by the third padlock probe as a partially double stranded domain, such that the third padlock probe hybridised to a different strand to the first or second padlock.

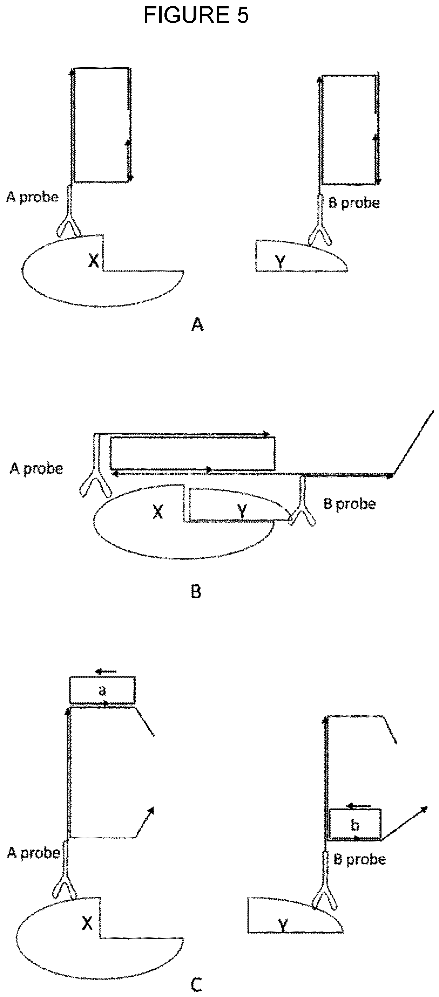

Similar arrangements of proximity probes with padlocks to detect members of a pair of target molecules, and an interaction between them, are depicted in **Figure 5****.** Part A shows a proximity probe pair, comprising first and second proximity probes (depicted as probes A and B), wherein each probe detects a different target molecule and wherein the nucleic acid domain of each proximity probe is hybridised to a first and second padlock probe-blocking oligonucleotide complex respectively. The target-binding regions of the first padlock probe are capable of hybridising to probe-binding sites in the second padlock probe. The second padlock probe thus serves as a ligation template for the first padlock probe. Whilst the blocking oligonucleotide attached to each padlock is shown having the configuration of Figure 1A, it could equally have the configuration of Figure 1B. The first padlock probe (as shown on probe A) is able to interact with the second padlock (as shown on probe B) when the two proximity probes are brought into proximity, for example when the respective target molecules are in an interaction. This may be achieved by competitive displacement of the blocking oligonucleotides from their respective padlocks by the respective targets of the padlocks or by use of one or two key oligonucleotides. This interaction between the two padlock probes after removal of the blocking oligonucleotide is depicted in Figure 5B, which also shows ligation of the first padlock probe using the second padlock probe as ligation template. Detection of the ligated first padlock probe allows detection of the interaction. If there is not an interaction or proximity between the target molecules, then it may be possible to detection an alternative or other interaction, as the ligation of the first padlock probe may be templated by other padlock probes carried by other proximity probes specific for other possible interaction partners for the first target molecule. Thus, by using other proximity probes (specific for different target molecules) each carrying a different "second" padlock probe, it may be possible to determine which of a number of different possible interaction partners have interacted with the first target molecule. Accordingly, the methods as described more generally herein may be used to study interaction networks.

To detect the individual target molecules, which have not interacted or which are not in close proximity, such that there is no interaction between the first and second padlocks (i.e. no interaction between the proximity probes) additional profiling padlock probes may be used, as depicted in Figure 5C. Such profiling padlocks (shown as padlocks "a" and "b") may be capable of hybridising to the first and second padlock probes, which may template the ligation of the profiling padlocks. In this manner, the individual target molecules may also be detected, and quantified. The detection rate of the first and second proximity probes can be adjusted by adding a suitable proportion of unphosphorylated profiling padlocks to facilitate the detection of target molecules at different expression levels. The profiling padlocks can be designed as shown in Figure 6, and the blocking oligonucleotide can be used for preventing non-specific ligation. The reactions depicted in Figures 5B and 5C may be performed simultaneously, for example the ligation reactions can be combined. When the proximity ligation has occurred, part of the ligation template will be occupied, so the profiling padlocks will not be able to be ligated. In other words, when the proximity ligation signal is generated, the individual signal will not show. When the two probes are apart (i.e. when the targets are not in proximity), the individual signals can be generated.

An alternative for detecting the individual target molecules (when not in proximity or interaction) is shown in Figure 5D. In this embodiment, the individual target molecules may be detected using the individual first and second padlock probes and their blocking oligonucleotides as gap-fill and ligation templates. As indicated above, the blocking oligonucleotide may comprise a detection sequence (e.g. a unique ID sequence or barcode), which lies in the gap sequence between the padlock probe-binding sites of the blocking oligonucleotide), the complement of which is incorporated into the circularised padlock probe by a gap-filling reaction (e.g. a gap-filling extension reaction or by hybridisation of a gap-filling oligonucleotide which is complementary to the gap sequence in the blocking oligonucleotide), before the padlock probe is ligated. Thus, in accordance with the principle above, padlock probes which remain bound to their respective blocking oligonucleotides may be distinguished from padlock probes which have become displaced from their blocking oligonucleotide, and interacted with the nucleic acid domain or padlock probe of another proximity probe (by virtue of the detection sequence incorporated into the subsequently gap-filled and circularised padlock probe, from its blocking oligonucleotide. These reactions may only take place if the target molecules have not interacted, or are not in proximity, as otherwise the proximity/interaction causes the blocking oligonucleotide to be displaced. Alternatively, the reactions may be carried out independently of one another. In this embodiment also, the detection rate of the individual first and second proximity probes may be adjusted by mixing with unphosphorylated gap-filling oligoncucleotides to facilitate the detection of target molecules at different dynamic ranges. In the case of the embodiment shown in Figure 5D, it is preferred to the ligation reactions of Figures 5B and 5D to be performed separately, as separate ligation reactions. However, with optimisation of ligation template length and blocking oligonucleotide length, it is possible to combine the two ligation steps into one step.

In another embodiment, the method provided is used in the context of a SNAIL-like arrangement for detection of a nucleic acid analyte using a pair of nucleic acid probes. In this case, the padlock probe is provided bound via its backbone to a first nucleic acid probe, which comprises at one end a padlock-binding domain (which hybridises to a complementary sequence in the padlock probe backbone) and at the other end an analyte-binding domain which is complementary to a sequence in the nucleic acid analyte. The target-binding domains of the padlock probe are complementary to probe-binding regions within the second nucleic acid probe. The second nucleic acid probe also comprises an analyte-binding domain. The analyte-binding domain of the second nucleic acid probe hybridises to a sequence adjacent to the sequence to which the analyte-binding domain of the first nucleic acid probe hybridises.

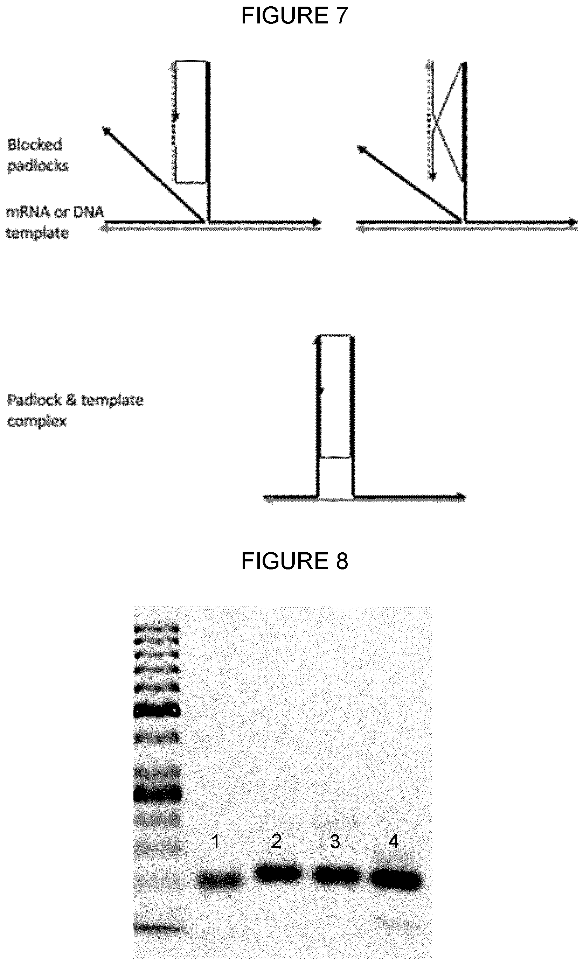

When the two nucleic acid probes in the pair both hybridise to the target analyte the probe-binding regions of the second nucleic acid probe displace the blocking oligonucleotide from the padlock probe, enabling circularisation of the padlock probe and subsequent detection as described above. Thus, in this instance, circularisation of the padlock probe requires hybridisation of both members of the nucleic acid probe pair to their analyte, and detection of the circularised padlock probe indicates the presence of the analyte. A schematic diagram showing this embodiment of the invention is presented in **Figure 7****.**

As detailed above, a kit is also provided herein. The kit provided comprises a padlock probe and a blocking oligonucleotide as described above. The padlock probe and blocking oligonucleotide may be provided separately, or together in complex. The kit may provide a single padlock probe/blocking oligonucleotide pair, or multiple such pairs.

The kit may further comprise any additional components for use in a detection reaction as discussed above or as routinely used. In particular embodiments, the kit comprises a key oligonucleotide (when the kit comprises multiple padlock probe/blocking oligonucleotide pairs, a matching key oligonucleotide may be provided for each pair). When the blocking oligonucleotide is enzymatically degraded, the enzyme used for the degradation reaction may be included in the kit. Suitable enzymes for this purpose are discussed above. In a particular embodiment the kit comprises uracil-DNA glycosylase and an endonuclease enzyme.

The kit may additionally comprise probes for an analyte, where the padlock probe is used in combination with another probe set as discussed above. The kit may for instance comprise one or proximity probes, in particular one or more proximity probe pairs. In line with the teaching above, a proximity probe pair may be provided with a padlock probe which is complementary to one probe in the pair via its backbone and the other probe in the set via its target binding sites. In this embodiment, the two proximity probes in the pair are generally provided separately in the kit (i.e. in separate containers). The padlock probe and blocking oligonucleotide may be provided separately to the proximity probe, or alternatively the padlock probe/blocking oligonucleotide complex may be provided pre-hybridised to the proximity probe complementary to the padlock probe backbone sequence.

In a particular embodiment, in line with the teaching above, the kit is provided with a pair of padlock probes and complementary blocking oligonucleotides, and a pair of proximity probes. In this case, each padlock probe hybridises to one proximity probe via its backbone and the other proximity probe via its target-binding regions, as detailed above. Again, the padlock probes and blocking oligonucleotides may be provided separately to the proximity probes, or each padlock probe/blocking oligonucleotide may be provided pre-hybridised to the proximity probe complementary to the padlock probe backbone sequence.

In another embodiment the padlock probe and blocking oligonucleotide are provided with a pair of nucleic acid probes for a SNAIL-like arrangement, as detailed above.

The kit may also be provided with standard components such as buffers, polymerases, nucleotides (e.g. dNTPs), detection oligonucleotides, etc.

The invention may be further understood by reference to the figures and non-limiting examples below:

### Figure Legends

Figure 1 shows the two possible basic structures of padlock probe/blocking oligonucleotide complexes. **A** shows a padlock probe (open black semi-circle) hybridised to a blocking oligonucleotide (straight grey arrow), arranged such that the 5' end (indicated by the arrow head) of the padlock probe hybridises to the 5' end of the blocking oligonucleotide. In this embodiment, the two ends of the padlock probe face towards each other, separated by a gap (the dotted section of the blocking oligonucleotide). **B** shows a padlock probe hybridised to a blocking oligonucleotide arranged such that the 5' end of the padlock probe hybridises to the 3' end of the blocking oligonucleotide. In this embodiment, the two ends of the padlock probe face away from each other.
Figure 2 is a basic schematic diagram showing the operation of a padlock probe/blocking oligonucleotide complex opened using a key oligonucleotide. As indicated the key oligonucleotide is the dashed arrow, the central black region of which is complementary for the central gap section of the blocking oligonucleotide, and the flanking grey regions of which are complementary to the padlock probe-binding sequences of the blocking oligonucleotide.
Figure 3 is a schematic diagram showing how a proximity probe pair, of which one probe comprises a padlock probe/blocking oligonucleotide complex hybridised to its nucleic acid domain, can be used in the context of a proximity ligation assay to detect an interaction between two target molecules (particularly an interaction between two proteins).
Figure 4 is a schematic diagram showing how a proximity probe pair, in which both proximity probes comprise a padlock probe/blocking oligonucleotide complex hybridised to the nucleic acid domain, can be used to detect two target molecules (particularly target proteins) and the interaction between them, and to calculate the amount of each target molecule which is interacting with its partner of interest.
Figure 5 is a schematic diagram showing how a proximity probe pair comprising first and second proximity probes, in which both proximity probes comprise a padlock probe/blocking oligonucleotide complex hybridised to the nucleic acid domain, can be used to detect two target molecules (particularly target proteins) and the interaction between them, and to calculate the amount of each target molecule which is interacting with its partner of interest. A) shows the two proximity probes, each with a hybridised padlock probe/blocking oligonucleotide complex. B) shows interaction of the respective target molecules, and consequent interaction between the first and second padlock probes hybridised respectively to the first and second proximity probes. C) shows detection of the individual proximity probes bound to their respective individual target molecules using separate profiling padlocks specific for the first and second padlock probes of the first and second proximity probes. D) shows detection of the individual proximity probes bound to their respective individual target molecules using their respective blocking oligonucleotides as templates for gap-filling and ligation reactions.
Figure 6 is a schematic diagram showing padlock probes comprising anchor sequences. Two padlock probe/blocking oligonucleotide complex structures are shown, corresponding to the structure shown in Fig. 1A (left) and Fig. 1B (right). In both cases the padlock probe comprises an anchor sequence at the 5' end of the 3' target-binding region (coloured grey), upstream of the section hybridised to the blocking oligonucleotide. This anchor sequence binds the target sequence, resulting in competitive displacement of the blocking oligonucleotide from the padlock probe by the target sequence.
Figure 7 is a schematic diagram showing padlock probes/blocking oligonucleotide complexes being used in the context of a SNAIL arrangement for detection of a nucleic acid analyte. Two padlock probe/blocking oligonucleotide complex structures are shown, corresponding to the structure shown in Fig. 1A (left) and Fig. 1B (right). In both cases the padlock probe is hybridised to a first nucleic acid probe via its backbone, while a second nucleic acid probe is also bound to the analyte, adjacent to the first nucleic acid probe (top). The second nucleic acid probe displaces the blocking oligonucleotide from the padlock probe, and hybridises to the target binding regions of the padlock probe itself (bottom).
Figure 8 shows an electrophoresis gel confirming the formation of a padlock probe - blocking oligonucleotide complex, in the configuration of Figure 1A, with different hybridization strength depending the length of hybridization sites on both ends of the padlocks (lanes 1 to 4 representing complexes with different length of blocking oligonucleotides with padlock probe binding sites each of 13, 19, 17 and 15 nt respectively and a gap sequence length of 12 nt). The size of complexes in each of lanes 1 to 4 are 105, 117, 113, and 109 nt respectively,

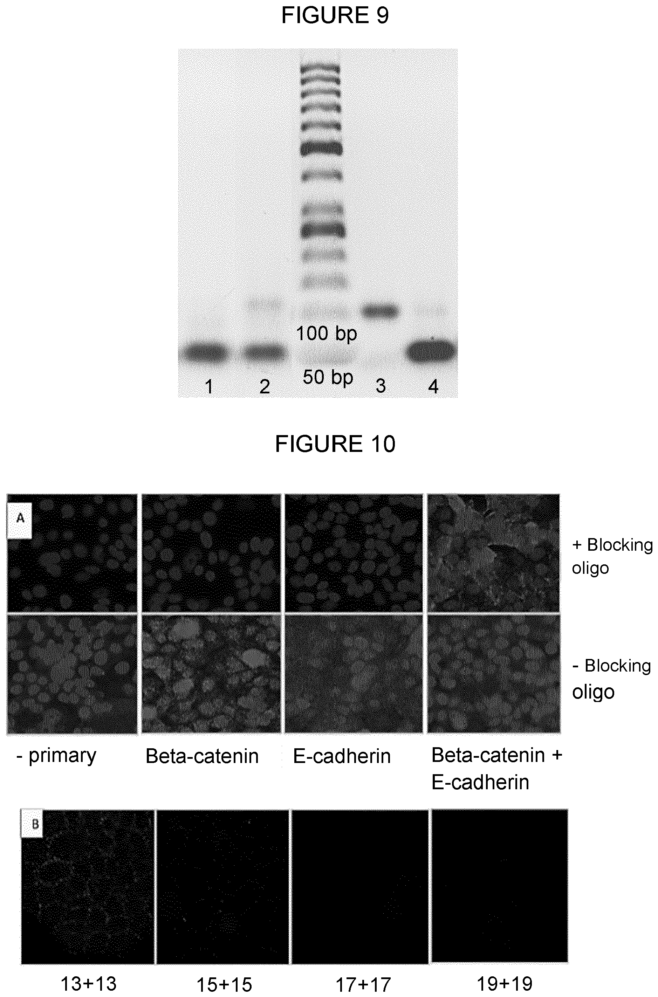

Figure 9 shows an electrophoresis gel showing padlock probe and blocking oligonucleotide complexes, and complexes which have been disrupted by a key oligonucleotide which is the full or partial reverse complement of the blocking oligonucleotide. Lane 1 band: free padlock (69 nt); lane 2 bands: free padlock (69 nt) band and blocking oligonucleotide (38 nt)-short key oligonucleotide (22 nt) complex (58 nt) band, two bands indistinguishable: lane 3 band: padlock probe-blocking oligo complex (105 nt); lane 4 bands: free padlock (69 nt) band and blocking oligonucleotide (38 nt)-long key oligonucleotide (34 nt) complex (72 nt) band, two bands indistinguishable from each other.
Figure 10 presents photomicrographs showing the results of a proximity ligation assay in a format as depicted in Figure 3, using a proximity probe pair comprising secondary antibodies directed against primary antibodies specific for the proteins beta-catenin and E-cadherin; (A) shows results using the proximity probe pair (with hybridised padlock probe) in the presence (+) (upper panel) and absence (-) (lower panel) of blocking oligonucleotide (13+13), in the following conditions: absence of primary antibody; primary antibody for beta-catenin only; primary antibody for E-cadherin only; both primary antibodies. (B) shows the effect of blocking oligonucleotides of different length, resulting in different hybridisation strengths: blocking oligonucleotides with padlock-probe binding regions 13+13, 15+15, 17+17, 19+19.

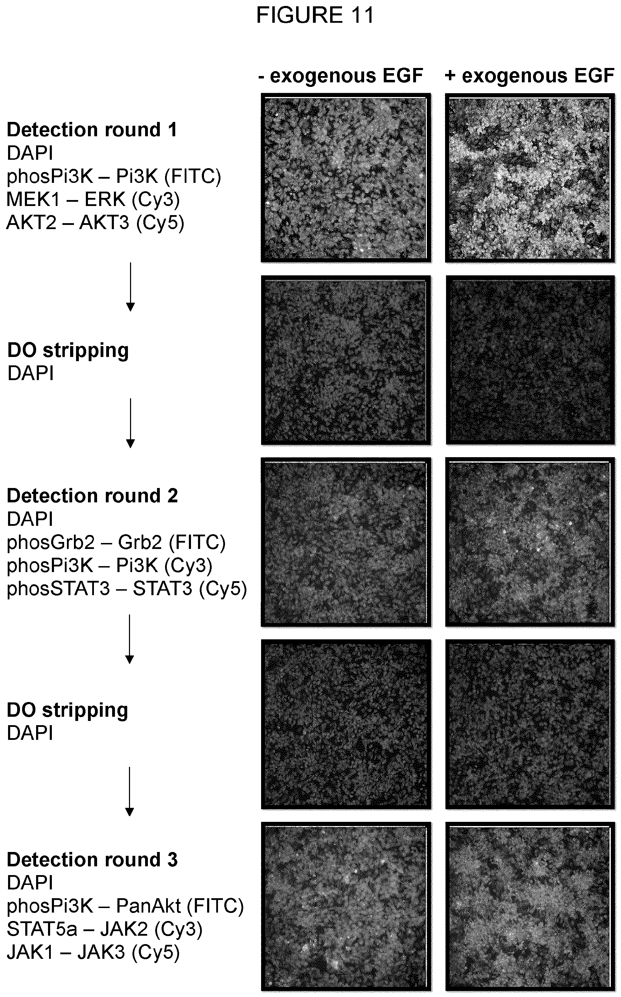

Figure 11 presents photomicrographs showing the results of multiplex in situ PLA performed on a human breast cancer cell line with and without EGF treatment. Detection was performed in three imaging rounds with stripping of the specific fluorescently-labelled detection probe between rounds. DAPI staining is shown in each image. Three isPLA reactions are detected per round using either a FITC, Cy3 or Cy5 filter. The isPLA assays target protein phosphorylation - protein or protein-protein interactions.

### Examples

### Example 1

Padlock probe-blocking oligonucleotide complexes having the format shown in Figure 1A was designed and prepared. In this design the blocking oligonucleotide has padlock probe-binding regions which occupy the target binding regions at the 5' and 3' ends of the padlock. The padlock probe binding regions are separated by a gap sequence in the blocking oligonucleotide. 4 different blocking oligonucleotides, each having a 12 nt gap sequence but with differing lengths of padlock probe binding regions at the 5' and 3' ends of the blocking oligonucleotide, were used to prepare 4 different complexes with the same 69 nt long padlock probe as follows:

| | Sequence |
|---|---|
| Padlock | ctcacccaactacatacaccaacA[?]CAAATCCGCGTGATAACGAAC[?]ACACACACAC Aacaacaccacca (SEQ ID NO. 1) |
| Ligation template | ACTCCCACTCCACTGGGTCTGGTCAAaaagttgggtgagtggtggtgttg (SEQ ID NO. 2) |
| Blocking oligo 13+13 | GT AGT TGG GTG AGC CGA TAA CAC TTT GGT GGT GTT GTT (SEQ ID NO. 3) |
| Blocking oligo 19+19 | GTGTATG TAG TTG GGT GAG CCG ATA ACA CTT TGG TGG TGT TGT TGTGTTG (SEQ ID NO. 4) |
| Blocking oligo 17+17 | GTATG TAG TTG GGT GAG CCG ATA ACA CTT TGG TGG TGT TGT TGTgt (SEQ ID NO. 5) |
| Blocking oligo 15+15 | ATG TAG TTG GGT GAG CCG ATA ACA CTT TGG TGG TGT TGT TGT (SEQ ID NO. 6) |
| Conjugati on oligo1 for padlock hybridizati on | TGGTTCGTTATCACGCGGATTTGTAAAAAA 3' azide (SEQ ID NO. 7) |
| Conjugati on oligo2 For ligation template hybridizati on | azideAAAAAATTGACCAGACCCAGTGGAGTGGGAGTC (SEQ ID NO. 8) |
| Detection oligo | /5Cy5/tacatacaccaacAC (SEQ ID NO. 9) |
| Key oligo short 22nt | caCC AAA GTG TTA TCG GCT Caa (SEQ ID NO. 10) |
| Key oligo long 34nt | caAC AAC ACC AAA GTG TTA TCG GCT CAA ACT Aca (SEQ ID NO. 11) |

| | |
|---|---|
| 1) 105 nt complex; padlock probe-binding regions of 13 nt each; 2) 117 nt complex; padlock probe-binding regions of 19 nt each; 3) 113 nt complex; padlock probe-binding regions of 17 nt each; 4) 109 nt complex; padlock probe-binding regions of 15 nt each. | |

To form the complexes the padlock probes and blocking oligonucleotides were mixed in PBS at a concentration of 100 nM each. The formation of the complexes was validated by electrophoresis on a 2% agarose gel, at 100v for 1 hour. The results are shown in Figure 8 (lanes 1-4 corresponding to complexes 1-4 above).

### Example 2

This example shows the process of blocking a padlock probe with a blocking oligonucleotide and "opening" or releasing the padlock probe with a key oligonucleotide, according to the schematic shown in Figure 2.

A padlock probe-blocking oligonucleotide complex prepared according to Example 1 was used (complex 3, with a 38 nt long blocking oligonucleotide (12 nt gap sequence and 13 nt long padlock probe binding ends). Two different key oligonucleotides were prepared, long (34 nt - reverse complement to the toehold region, plus 12nt on each site which are reverse complement to padlock hybridization site) and short (22 nt - reverse complement to the toehold region ,plus 6nt on each site which are reverse complement to padlock hybridization site).

The padlock probe-blocking oligonucleotide complexes were contacted separately with each of the long and short key oligonucleotides (100 nM in PBS for 30min at room temperature). The resulting mixture was subjected to electrophoresis (2% agarose gel, 100v, 1 hour) and the results are shown in Figure 9, to validate formation and disruption of the complexes.

Lane1 band: free padlock (69 nt); lane 3 band: padlock & blocking oligo complex (105 nt); lane 2 bands: free padlock (69 nt) band and blocking oligo (38 nt) & short key oligo (22 nt) complex (58 nt) band, two bands indistinguishable, lane 4 bands: free padlock (69 nt) band and blocking oligo (38 nt) & long key oligo (34 nt) complex (70 nt) band, two bands indistinguishable from each other.

Lanes 2 and 4 clearly shown a band the size of the free padlock, confirming that both key oligonucleotides were able to displace the blocking oligonucleotide and release free padlock probe.

### Example 3

This example shows the application of the padlock probe blocking strategy in a proximity ligation assay, according to the format shown in Figure 3.

The blocking oligonucleotide is hybridized to the padlock probe on the first proximity probe (probe A). The conjugated nucleic acid domain of the second proximity probe (probe B) acts as a ligation template for the padlock probe (i.e. it is the target molecule of the padlock probe). This ligation template is not able to hybridize to the padlock probe on probe A when in solution, due to the presence of the blocking oligonucleotide, which blocks the target binding regions of the padlock probe. When the probe A and B are in close proximity, namely when their respective targets are in close proximity (e.g. when they are in an interaction/complex as depicted in Figure 3) and the proximity probes have both bound to their respective targets, the high local concentration of ligation template can compete away the blocking oligonucleotide, and enable the ligation of the padlock.

The validation of the design was performed on MCF-7 cells, using a high concentration of proximity probes (conjugated secondary antibodies), probe A: anti-Mouse, with padlocks hybridized; probe B: anti-Rabbit with ligation templates (24 nt hybridization site, 12 nt on each end), 50 nM each). After incubation the fixed MCF-7 cells with (one or both primary antibody for Beta-caternin and E-cadherin) or without the primary antibodies (-primary) and washing, the secondary antibodies with (1 uM blocking oligos, 13 nt hybridization site on both ends) or without blocking oligos were applied to the MCF-7 cells. The padlocks were ligated by T4 ligase to generate single stranded DNA circles and followed by rolling cycle amplification using Phi29 as described in Klaesson et al., Scientific Reports, (2018) 8:5400.

The results are shown in Figure 10A.

After ligation and rolling cycle amplification, the panel with no blocking oligonucleotides generated high background for all conditions, suggesting the formation of probe A and B complexes in solution. For the upper panel with blocking oligonucleotides, only the condition with both primary antibodies generated proximity signals representing the interaction of Beta-catenin and E-cadherin. This demonstrates that the blocking oligonucleotide can prevent the formation of padlock and ligation template complex in solution. When the probe A and B bind in close proximity, the blocking oligonucleotide can be competed away. The signal here results from the rolling cycle amplification products, representing the close proximity of Beta-catenin and E-cadherin detected by Cy5 labelled detection oligonucleotides, the nuclei were stained with DAPI.

Figure 10B shows the results of an experiment conducted to show that the proximity competition efficiency of ligation templates with blocking oligonucleotides can be affected by the length of the blocking oligonucleotides. Four blocking oligonucleotides with different hybridization strength were tested in MCF-7 cells with the same conditions as described above. The blocking oligonucleotides and padlock probes were as described in Example 1 and depicted in Figure 1A. Thus, for example, the blocking oligonucleotide denoted as 13+13 in Figure 10B means the hybridization site (i.e. padlock probe binding region) length on each end of the blocking oligonucleotide is 13 nt. Only the 13+13 blocking oligonucleotide, having a similar hybridization length as the ligation template on each end of the padlock probe, generated good proximity signals. This suggests that for longer blocking oligonucleotides, key oligonucleotides may be required or beneficial to remove the blocking oligonucleotide efficiently. It will be appreciated, however, that the design of the padlocks and blocking oligonucleotides may be optimised in each case to improve efficiency.

### Example 4

In situ PLA probes using the set-up illustrated in Figure 3 were generated to target protein-protein interactions or protein-protein phosphorylation targets. Each isPLA probe set contains a unique oligonucleotide sequence that is complimentary to a fluorescent-conjugated detection oligonucleotide. The detection oligos are conjugated to either FITC, Cy3 or Cy5. The isPLA probes target phosphorylated Pi3k, Grb2 and STAT3, and the interactions MEK1 - ERK, AKT2 - AKT3, phospPl3K - panAKT, STAT5a- JAK2 and JAK1 - JAK3.

The isPLA probes were pooled and added to fixed human breast cancer cells that was either stimulated with EGF or not. EGF stimulation is expected to increase the expression of many of the phosphorylations and interactions measured.

Following probe incubation, the padlocks were circularised by ligation, subjected to RCA to generate RCA products and detected with fluorescently labelled detection probes. Detection was performed in three rounds (see Figure 11). In round one, three detection oligos plus DAPI were added to detect phosphorylated Pi3K, MEK1 - ERK interaction and AKT2 - AKT3 interaction. isPLA signal is observed as punctate dots in the images (Figure 11). Next, the detection oligos were stripped and new detection oligos were added targeting phosphorylated Grb2, phosphorylated Pi3K and phosphorylated STAT3. The same procedure was repeated again to detect phosphorylated Pi3K - PanAKT interaction, STAT5a - JAK2 interaction and JAK1 - JAK3 interaction. As expected, higher isPLA signal was observed in the EGF stimulated cells compared to the unstimulated cells.

## Claims

1. A method of detecting a target nucleic acid molecule in a sample, the method comprising:
(i) contacting the sample with a complex comprising a padlock probe and a blocking oligonucleotide,
wherein the padlock probe comprises at its 5' and 3' ends target-binding regions which are complementary to probe-binding sites in the target nucleic acid molecule,
and the blocking oligonucleotide comprises hybridisation sites which are complementary to the target-binding regions of the padlock probe;
wherein the blocking oligonucleotide comprises a sequence which is not complementary to the padlock probe;
wherein in the complex the hybridisation sites of the blocking oligonucleotide are hybridised to the target-binding regions of the padlock probe, in an arrangement whereby the 3' and 5' ends of the padlock probe cannot be ligated to each other;
(ii) causing removal of the blocking oligonucleotide from the complex such that the target-binding regions of the padlock probe hybridise to the probe-binding sites of the target nucleic acid molecule in an arrangement allowing direct or indirect ligation of the target-binding regions to each other;
(iii) directly or indirectly ligating the target-binding regions of the padlock probe to each other, thereby circularising the padlock probe;
(iv) amplifying the circularised padlock probe or a part thereof; and
(v) detecting the amplification product of (iv) in order to detect the target nucleic acid sequence.

2. The method of claim 1, wherein the blocking oligonucleotide comprises at least one toehold region which does not hybridise to the padlock probe and is long enough to allow a second oligonucleotide to invade the complex and displace the blocking oligonucleotide from the padlock probe.

3. The method of claim 1, wherein the padlock probe comprises an anchor sequence that is a continuation of a target-binding sequence within the padlock probe and is complementary to the target sequence in the target nucleic acid molecule.

4. The method of any one of claims 1 to 3, wherein in the complex a gap is present between the target-binding regions of the padlock probe, optionally wherein the gap is at least 1, 2, 3, 4, 5 or 6 nucleotides long.

5. The method of any one of claims 1 to 4, wherein in step (ii) the blocking oligonucleotide is competitively displaced from the padlock probe by the target nucleic acid molecule.

6. The method of any one of claims 1 to 4, wherein in step (ii) the blocking oligonucleotide is removed from the padlock probe using a key oligonucleotide complementary to the blocking oligonucleotide, wherein the blocking oligonucleotide hybridises more strongly to the key oligonucleotide than to the padlock probe, optionally wherein the key oligonucleotide is 100 % complementary to the gap sequence in the blocking oligonucleotide, and less than 100 % complementary to one or both hybridisation sites in the blocking oligonucleotide.

7. The method of any one of claims 1 to 4, wherein in step (ii) the blocking oligonucleotide is removed by enzymatic digestion or photo-cleavage, optionally wherein:
(a) the blocking oligonucleotide is a DNA molecule comprising one or more restriction sites not present in the padlock probe or the target nucleic acid, and is digested using one or more restriction enzymes which recognise the restriction sites;
(b) the blocking oligonucleotide is a DNA molecule comprising one or more uridine residues, and is digested using uracil-DNA glycosylase and an endonuclease; or
(c) the blocking oligonucleotide comprises a photo-cleavable linker.

8. The method of any one of claims 1 to 7, wherein the target nucleic acid molecule is a target analyte, is generated from a target analyte or is a reporter for a target analyte, optionally wherein the analyte is a protein and the target nucleic acid molecule is a reporter comprised within a detection probe for the protein, preferably wherein the detection probe comprises an antibody specific for the protein conjugated to the target nucleic acid molecule.

9. The method of any one of claims 1 to 8, wherein:
(a) the target nucleic acid molecule is the nucleic acid domain of a proximity probe;
(b) the circularised padlock probe is amplified by rolling circle amplification; and/or
(c) the padlock probe comprises a detection sequence, wherein the detection sequence allows the padlock probe, or an amplicon or reverse complement copy thereof to be detected, optionally wherein the detection sequence is a binding site for a detection oligonucleotide, or comprises a barcode sequence, optionally wherein the detection sequence or its reverse complement is detected using a detection oligonucleotide linked to a detection moiety, optionally wherein the detection moiety is a bead, a fluorescent or colorimetric label, a dye, or an enzyme substrate.

10. The method of any one of claims 1 to 9, for detecting a target molecule in the sample, wherein the sample is contacted with a pair of proximity probes specific for the target molecule, each proximity probe comprising a nucleic acid domain,
wherein the nucleic acid domain of at least one proximity probe is hybridised to a padlock probe-blocking oligonucleotide complex,
and the target-binding regions of the padlock probe hybridise to probe-binding sites located in either the other nucleic acid domain or to another padlock probe hybridised to the other nucleic acid domain.

11. The method of any one of claims 1 to 9, for detecting an interaction between two target molecules in the sample, wherein the sample is contacted with a first proximity probe specific for the first target molecule and a second proximity probe specific for the second target molecule, each proximity probe comprising a nucleic acid domain,
wherein the nucleic acid domain of at least one proximity probe is hybridised to a padlock probe-blocking oligonucleotide complex,
and the target-binding regions of the padlock probe hybridise to probe-binding sites located in either the other nucleic acid domain or to another padlock probe hybridised to the other nucleic acid domain.

12. The method of claim 11, for detecting two target molecules in a sample and the interaction between them, comprising:
(i) contacting the sample with a first proximity probe for detection of the first target molecule and a second proximity probe for detection of the second target molecule, wherein said proximity probes each comprise a binding domain and a nucleic acid domain, and said first and second proximity probe together form a proximity probe pair for detection of the interaction between the two target molecules;
wherein the nucleic acid domain of the first proximity probe is hybridised to a first padlock probe-blocking oligonucleotide complex, and the nucleic acid domain of the second detection probe is hybridised to a second padlock probe-blocking oligonucleotide complex;
wherein the target-binding regions of the first padlock probe are capable of hybridising to probe-binding sites in the nucleic acid domain of the second proximity probe or in the second padlock probe, and the target-binding regions of the second padlock probe are capable of hybridising to probe-binding sites in the nucleic acid domain of the first proximity probe, or in the first padlock probe;
and wherein the blocking oligonucleotides are displaced from the padlock probes when the padlock probes are in close proximity to their target nucleic acid molecules, such that upon binding of the first and second proximity probes to interacting target molecules the padlock probes hybridise to their respective target nucleic acid molecules, while a padlock probe hybridised to a detection probe bound to a non-interacting target molecule remains in complex with its blocking oligonucleotide;
(ii) performing a gap-filling and ligation reaction, thereby generating circularised ligation products, wherein the ligation product from a padlock probe in complex with its blocking oligonucleotide comprises a barcode sequence from the blocking oligonucleotide, such that circularisation of the padlock probes generates distinct products indicating interacting and non-interacting target molecules;
(iii) detecting the circularisation products and their relative levels, thereby determining the proportion of each target molecule interacting with the other, optionally wherein the circularisation products are detected by sequencing or qPCR.

13. The method of claim 11 or 12, wherein the two target molecules are two proteins, or a protein and a nucleic acid molecule.

14. A kit for detecting a target nucleic acid molecule in a sample, comprising a padlock probe and a blocking oligonucleotide as defined in any one of claims 1 to 4, 8 or 9.

15. The kit of claim 24, further comprising a proximity probe as defined in claim 7, optionally further comprising a pair of padlock probes, a pair of blocking oligonucleotides, and a pair of proximity probes as defined in claim 12.

## Patentansprüche

1. Verfahren zum Nachweisen eines Zielnukleinsäuremoleküls in einer Probe, wobei das Verfahren umfasst:
(i) Bringen der Probe in Kontakt mit einem Komplex, der eine Padlock-Sonde und ein blockierendes Oligonukleotid umfasst,
wobei die Padlock-Sonde an dem 5'- und dem 3'-Ende Zielbindungsregionen umfasst, die zu Sondenbindungsstellen in dem Zielnukleinsäuremolekül komplementär sind,
und das blockierende Oligonukleotid Hybridisierungsstellen umfasst, die zu den Zielbindungsregionen der Padlock-Sonde komplementär sind;
wobei das blockierende Oligonukleotid eine Sequenz umfasst, die nicht komplementär zu der Padlock-Sonde ist;
wobei in dem Komplex die Hybridisierungsstellen des blockierenden Oligonukleotids in einer Anordnung, durch die das 3'- und das 5'-Ende der Padlock-Sonde nicht miteinander ligiert werden können, an die Zielbindungsregionen der Padlock-Sonde hybridisiert sind;
(ii) Bewirken einer Entfernung des blockierenden Oligonukleotids aus dem Komplex, sodass die Zielbindungsregionen der Padlock-Sonde in einer Anordnung, die eine direkte oder indirekte Ligation der Zielbindungsregionen miteinander ermöglicht, an die Sondenbindungsstellen des Zielnukleinsäuremoleküls hybridisieren;
(iii) direktes oder indirektes Ligieren der Zielbindungsregionen der Padlock-Sonde miteinander, wodurch die Padlock-Sonde zirkularisiert wird;
(iv) Amplifizieren der zirkularisierten Padlock-Sonde oder eines Teils davon; und
(v) Nachweisen des Amplifikationsprodukts aus (iv) zum Nachweisen der Zielnukleinsäuresequenz.

2. Verfahren nach Anspruch 1, wobei das blockierende Oligonukleotid mindestens eine Toehold-Region umfasst, die nicht an die Padlock-Sonde hybridisiert und lang genug ist, um es einem zweiten Oligonukleotid zu ermöglichen, in den Komplex einzudringen und das blockierende Oligonukleotid von der Padlock-Sonde weg zu verdrängen.

3. Verfahren nach Anspruch 1, wobei die Padlock-Sonde eine Ankersequenz umfasst, die eine Fortsetzung einer Zielbindungssequenz innerhalb der Padlock-Sonde ist und komplementär zu der Zielsequenz in dem Zielnukleinsäuremolekül ist.

4. Verfahren nach einem der Ansprüche 1 bis 3, wobei in dem Komplex ein Spalt zwischen den Zielbindungsregionen der Padlock-Sonde vorhanden ist, wobei optional der Spalt mindestens 1, 2, 3, 4, 5 oder 6 Nukleotide lang ist.

5. Verfahren nach einem der Ansprüche 1 bis 4, wobei in Schritt (ii) das blockierende Oligonukleotid durch das Zielnukleinsäuremolekül kompetitiv von der Padlock-Sonde weg verdrängt wird.

6. Verfahren nach einem der Ansprüche 1 bis 4, wobei in Schritt (ii) das blockierende Oligonukleotid unter Verwendung eines Schlüsseloligonukleotids, das zu dem blockierenden Oligonukleotid komplementär ist, von der Padlock-Sonde entfernt wird, wobei das blockierende Oligonukleotid stärker an das Schlüsseloligonukleotid hybridisiert als an die Padlock-Sonde, wobei optional das Schlüsseloligonukleotid zu 100 % komplementär zu der Spaltsequenz in dem blockierenden Oligonukleotid ist und zu weniger als 100 % komplementär zu einer oder beiden Hybridisierungsstellen in dem blockierenden Oligonukleotid ist.

7. Verfahren nach einem der Ansprüche 1 bis 4, wobei in Schritt (ii) das blockierende Oligonukleotid durch enzymatischen Verdau oder Photospaltung entfernt wird, wobei optional:
(a) das blockierende Oligonukleotid ein DNA-Molekül ist, das eine oder mehrere Restriktionsstellen umfasst, die nicht in der Padlock-Sonde oder der Zielnukleinsäure vorhanden sind, und unter Verwendung eines oder mehrerer Restriktionsenzyme verdaut wird, die die Restriktionsstellen erkennen;
(b) das blockierende Oligonukleotid ein DNA-Molekül ist, das einen oder mehrere Uridinreste umfasst und unter Verwendung von Uracil-DNA-Glykosylase und einer Endonuklease verdaut wird; oder
(c) das blockierende Oligonukleotid einen photospaltbaren Linker umfasst.

8. Verfahren nach einem der Ansprüche 1 bis 7, wobei das Zielnukleinsäuremolekül ein Zielanalyt ist, aus einem Zielanalyten erzeugt wird oder ein Reporter für einen Zielanalyten ist, wobei optional der Analyt ein Protein ist und das Zielnukleinsäuremolekül ein Reporter ist, der in einer Nachweissonde für das Protein umfasst ist, wobei vorzugsweise die Nachweissonde einen Antikörper umfasst, der spezifisch für das an das Zielnukleinsäuremolekül konjugierte Protein ist.

9. Verfahren nach einem der Ansprüche 1 bis 8, wobei:
(a) das Zielnukleinsäuremolekül die Nukleinsäuredomäne einer Proximity-Sonde ist;
(b) die zirkularisierte Padlock-Sonde durch Rolling-Circle-Amplifikation amplifiziert wird; und/oder
(c) die Padlock-Sonde eine Nachweissequenz umfasst, wobei die Nachweissequenz den Nachweis der Padlock-Sonde oder eines Amplikons oder einer reversen Komplementärkopie davon ermöglicht, wobei optional die Nachweissequenz eine Bindungsstelle für ein Nachweisoligonukleotid ist oder eine Barcode-Sequenz umfasst, wobei optional die Nachweissequenz oder deren reverses Komplement unter Verwendung eines Nachweisoligonukleotids nachgewiesen wird, das mit einer Nachweisgruppe verbunden ist, wobei optional die Nachweisgruppe ein Mikrokügelchen, eine fluoreszierende oder kolorimetrische Markierung, ein Farbstoff oder ein Enzymsubstrat ist.

10. Verfahren nach einem der Ansprüche 1 bis 9, zum Nachweisen eines Zielmoleküls in der Probe, wobei die Probe mit einem Paar von Proximity-Sonden in Kontakt gebracht wird, die für das Zielmolekül spezifisch sind, wobei jede Proximity-Sonde eine Nukleinsäuredomäne umfasst,
wobei die Nukleinsäuredomäne von mindestens einer Proximity-Sonde an einen Komplex aus einer Padlock-Sonde und einem blockierenden Oligonukleotid hybridisiert wird,
und die Zielbindungsregionen der Padlock-Sonde an Sondenbindungsstellen hybridisieren, die sich entweder in der anderen Nukleinsäuredomäne oder an einer anderen Padlock-Sonde befinden, die an die andere Nukleinsäuredomäne hybridisiert ist.

11. Verfahren nach einem der Ansprüche 1 bis 9, zum Nachweisen einer Interaktion zwischen zwei Zielmolekülen in der Probe, wobei die Probe mit einer ersten Proximity-Sonde, die für das erste Zielmolekül spezifisch ist, und einer zweiten Proximity-Sonde, die für das zweite Zielmolekül spezifisch ist, in Kontakt gebracht wird, wobei jede Proximity-Sonde eine Nukleinsäuredomäne umfasst,
wobei die Nukleinsäuredomäne von mindestens einer Proximity-Sonde an einen Komplex aus einer Padlock-Sonde und einem blockierenden Oligonukleotid hybridisiert wird,
und die Zielbindungsregionen der Padlock-Sonde an Sondenbindungsstellen hybridisieren, die sich in entweder der anderen Nukleinsäuredomäne oder an einer anderen Padlock-Sonde befinden, die an die andere Nukleinsäuredomäne hybridisiert ist.

12. Verfahren nach Anspruch 11, zum Nachweisen von zwei Zielmolekülen in einer Probe und der Interaktion dazwischen, umfassend:
(i) Bringen der Probe in Kontakt mit einer ersten Proximity-Sonde zum Nachweis des ersten Zielmoleküls und einer zweiten Proximity-Sonde zum Nachweis des zweiten Zielmoleküls, wobei die Proximity-Sonden jeweils eine Bindungsdomäne und eine Nukleinsäuredomäne umfassen, und die erste und die zweite Proximity-Sonde zusammen ein Proximity-Sondenpaar zum Nachweis der Interaktion zwischen den zwei Zielmolekülen bilden;
wobei die Nukleinsäuredomäne der ersten Proximity-Sonde an einen ersten Komplex aus einer Padlock-Sonde und einem blockierenden Oligonukleotid hybridisiert wird und die Nukleinsäuredomäne der zweiten Nachweissonde an einen zweiten Komplex aus einer Padlock-Sonde und einem blockierenden Oligonukleotid hybridisiert wird;
wobei die Zielbindungsregionen der ersten Padlock-Sonde in der Lage sind, an Sondenbindungsstellen in der Nukleinsäuredomäne der zweiten Proximity-Sonde oder in der zweiten Padlock-Sonde zu hybridisieren, und die Zielbindungsregionen der zweiten Padlock-Sonde in der Lage sind, an Sondenbindungsstellen in der Nukleinsäuredomäne der ersten Proximity-Sonde oder in der ersten Padlock-Sonde zu hybridisieren;
und wobei die blockierenden Oligonukleotide von den Padlock-Sonden weg verdrängt werden, wenn sich die Padlock-Sonden in unmittelbarer Nähe ihrer Zielnukleinsäuremoleküle befinden, sodass bei Bindung der ersten und zweiten Proximity-Sonde an interagierende Zielmoleküle die Padlock-Sonden an ihre jeweiligen Zielnukleinsäuremoleküle hybridisieren, während eine an eine Nachweissonde hybridisierte Padlock-Sonde, die an ein nicht-interagierendes Zielmolekül gebunden ist, im Komplex mit ihrem blockierenden Oligonukleotid verbleibt;
(ii) Durchführen einer Spaltfüllungs- und Ligationsreaktion, wodurch zirkularisierte Ligationsprodukte erzeugt werden, wobei das Ligationsprodukt einer Padlock-Sonde im Komplex mit ihrem blockierenden Oligonukleotid eine Barcode-Sequenz aus dem blockierenden Oligonukleotid umfasst, sodass die Zirkularisierung der Padlock-Sonden unterschiedliche Produkte erzeugt, die interagierende und nicht interagierende Zielmoleküle anzeigen;
(iii) Nachweisen der Zirkularisierungsprodukte und der relativen Konzentrationen davon, wodurch der Anteil jedes Zielmoleküls bestimmt wird, das mit dem anderen interagiert, wobei optional die Zirkularisierungsprodukte durch Sequenzierung oder qPCR nachgewiesen werden.

13. Verfahren nach Anspruch 11 oder 12, wobei es sich bei den zwei Zielmolekülen um zwei Proteine oder um ein Protein und ein Nukleinsäuremolekül handelt.

14. Kit zum Nachweisen eines Zielnukleinsäuremoleküls in einer Probe, umfassend eine Padlock-Sonde und ein blockierendes Oligonukleotid nach einem der Ansprüche 1 bis 4, 8 oder 9.

15. Kit nach Anspruch 24, weiter umfassend eine Proximity-Sonde nach Anspruch 7, optional weiter umfassend ein Paar von Padlock-Sonden, ein Paar von blockierenden Oligonukleotiden und ein Paar von Proximity-Sonden nach Anspruch 12.

## Revendications

1. Procédé de détection d'une molécule d'acide nucléique cible dans un échantillon, le procédé comprenant :
(i) la mise en contact de l'échantillon avec un complexe comprenant une sonde cadenas et un oligonucléotide de blocage,
dans lequel la sonde cadenas comprend, à ses extrémités 5' et 3', des régions de liaison à la cible qui sont complémentaires de sites de liaison à la sonde dans la molécule d'acide nucléique cible,
et l'oligonucléotide de blocage comprend des sites d'hybridation qui sont complémentaires des régions de liaison à la cible de la sonde cadenas ;
dans lequel l'oligonucléotide de blocage comprend une séquence qui n'est pas complémentaire de la sonde cadenas ;
dans lequel, dans le complexe, les sites d'hybridation de l'oligonucléotide de blocage sont hybridés aux régions de liaison à la cible de la sonde cadenas, dans un agencement tel que les extrémités 3' et 5' de la sonde cadenas ne peuvent pas se ligaturer l'une à l'autre ;
(ii) le fait de provoquer le retrait de l'oligonucléotide de blocage du complexe de telle sorte que les régions de liaison à la cible de la sonde cadenas s'hybrident aux sites de liaison à la sonde de la molécule d'acide nucléique cible dans un agencement permettant la ligature directe ou indirecte des régions de liaison à la cible l'une à l'autre ;
(iii) la ligature directe ou indirecte des régions de liaison à la cible de la sonde cadenas l'une à l'autre, pour ainsi entraîner la circularisation de la sonde cadenas ;
(iv) l'amplification de la sonde cadenas circularisée ou d'une partie de celle-ci ; et
(v) la détection du produit d'amplification de (iv) afin de détecter la séquence d'acide nucléique cible.

2. Procédé selon la revendication 1, dans lequel l'oligonucléotide de blocage comprend au moins une région d'accrochage qui ne s'hybride pas à la sonde cadenas et est suffisamment longue pour permettre à un deuxième oligonucléotide d'envahir le complexe et de déplacer l'oligonucléotide de blocage à partir de la sonde cadenas.

3. Procédé selon la revendication 1, dans lequel la sonde cadenas comprend une séquence d'ancrage qui est un prolongement d'une séquence de liaison à la cible au sein de la sonde cadenas et est complémentaire de la séquence cible dans la molécule d'acide nucléique cible.

4. Procédé selon l'une quelconque des revendications 1 à 3, dans lequel, dans le complexe, une brèche est présente entre les régions de liaison à la cible de la sonde cadenas, facultativement dans lequel la brèche présente une longueur d'au moins 1, 2, 3, 4, 5 ou 6 nucléotides.

5. Procédé selon l'une quelconque des revendications 1 à 4, dans lequel, à l'étape (ii), l'oligonucléotide de blocage est déplacé de manière compétitive depuis la sonde cadenas par la molécule d'acide nucléique cible.

6. Procédé selon l'une quelconque des revendications 1 à 4, dans lequel, à l'étape (ii), l'oligonucléotide de blocage est retiré de la sonde cadenas en utilisant un oligonucléotide clé complémentaire de l'oligonucléotide de blocage, dans lequel l'oligonucléotide de blocage s'hybride plus fortement à l'oligonucléotide clé qu'à la sonde cadenas, facultativement dans lequel l'oligonucléotide clé est complémentaire à 100 % de la séquence de brèche dans l'oligonucléotide de blocage, et complémentaire à moins de 100 % de l'un ou des deux sites d'hybridation dans l'oligonucléotide de blocage.

7. Procédé selon l'une quelconque des revendications 1 à 4, dans lequel, à l'étape (ii), l'oligonucléotide de blocage est retiré par digestion enzymatique ou photoclivage, facultativement dans lequel :
(a) l'oligonucléotide de blocage est une molécule d'ADN comprenant un ou plusieurs sites de restriction non présents dans la sonde cadenas ou l'acide nucléique cible, et est digéré en utilisant une ou plusieurs enzymes de restriction qui reconnaissent les sites de restriction ;
(b) l'oligonucléotide de blocage est une molécule d'ADN comprenant un ou plusieurs résidus d'uridine, et est digéré en utilisant une uracile-ADN glycosylase et une endonucléase ; ou
(c) l'oligonucléotide de blocage comprend un lieur photoclivable.

8. Procédé selon l'une quelconque des revendications 1 à 7, dans lequel la molécule d'acide nucléique cible est un analyte cible, est générée à partir d'un analyte cible ou est un rapporteur pour un analyte cible, facultativement dans lequel l'analyte est une protéine et la molécule d'acide nucléique cible est un rapporteur compris au sein d'une sonde de détection pour la protéine, préférablement dans lequel la sonde de détection comprend un anticorps spécifique de la protéine conjuguée à la molécule d'acide nucléique cible.

9. Procédé selon l'une quelconque des revendications 1 à 8, dans lequel :
(a) la molécule d'acide nucléique cible est le domaine d'acide nucléique d'une sonde de proximité ;
(b) la sonde cadenas circularisée est amplifiée par amplification en cercle roulant ; et/ou
(c) la sonde cadenas comprend une séquence de détection, dans lequel la séquence de détection permet de détecter la sonde cadenas, ou un amplicon ou une copie complémentaire inverse de celle-ci, facultativement dans lequel la séquence de détection est un site de liaison pour un oligonucléotide de détection, ou comprend une séquence code-barres, facultativement dans lequel la séquence de détection ou son complément inverse est détecté(e) en utilisant un oligonucléotide de détection lié à une fraction de détection, facultativement dans lequel la fraction de détection est une bille, un marqueur fluorescent ou colorimétrique, un colorant ou un substrat enzymatique.

10. Procédé selon l'une quelconque des revendications 1 à 9, pour détecter une molécule cible dans l'échantillon, dans lequel l'échantillon est mis en contact avec une paire de sondes de proximité spécifiques de la molécule cible, chaque sonde de proximité comprenant un domaine d'acide nucléique,
dans lequel le domaine d'acide nucléique d'au moins une sonde de proximité est hybridé à un complexe sonde cadenas-oligonucléotide de blocage,
et les régions de liaison à la cible de la sonde cadenas s'hybrident à des sites de liaison à la sonde situés soit dans l'autre domaine d'acide nucléique soit sur une autre sonde cadenas hybridée à l'autre domaine d'acide nucléique.

11. Procédé selon l'une quelconque des revendications 1 à 9, pour détecter une interaction entre deux molécules cibles dans l'échantillon, dans lequel l'échantillon est mis en contact avec une première sonde de proximité spécifique de la première molécule cible et une deuxième sonde de proximité spécifique de la deuxième molécule cible, chaque sonde de proximité comprenant un domaine d'acide nucléique,
dans lequel le domaine d'acide nucléique d'au moins une sonde de proximité est hybridé à un complexe sonde cadenas-oligonucléotide de blocage,
et les régions de liaison à la cible de la sonde cadenas s'hybrident à des sites de liaison à la sonde situés soit dans l'autre domaine d'acide nucléique soit sur une autre sonde cadenas hybridée à l'autre domaine d'acide nucléique.

12. Procédé selon la revendication 11, pour détecter deux molécules cibles dans un échantillon et l'interaction entre elles, comprenant :
(i) la mise en contact de l'échantillon avec une première sonde de proximité pour la détection de la première molécule cible et une deuxième sonde de proximité pour la détection de la deuxième molécule cible, dans lequel lesdites sondes de proximité comprennent chacune un domaine de liaison et un domaine d'acide nucléique, et lesdites première et deuxième sondes de proximité forment ensemble une paire de sondes de proximité pour la détection de l'interaction entre les deux molécules cibles ;
dans lequel le domaine d'acide nucléique de la première sonde de proximité est hybridé à un premier complexe sonde cadenas-oligonucléotide de blocage, et le domaine d'acide nucléique de la deuxième sonde de détection est hybridé à un deuxième complexe sonde cadenas-oligonucléotide de blocage ;
dans lequel les régions de liaison à la cible de la première sonde cadenas sont aptes à s'hybrider à des sites de liaison à la sonde dans le domaine d'acide nucléique de la deuxième sonde de proximité ou dans la deuxième sonde cadenas, et les régions de liaison à la cible de la deuxième sonde cadenas sont aptes à s'hybrider à des sites de liaison à la sonde dans le domaine d'acide nucléique de la première sonde de proximité, ou dans la première sonde cadenas ;
et dans lequel les oligonucléotides de blocage sont déplacés à partir des sondes cadenas lorsque les sondes cadenas se situent à proximité de leurs molécules d'acide nucléique cibles, de telle sorte que, lors de la liaison des première et deuxième sondes de proximité à des molécules cibles en interaction, les sondes cadenas s'hybrident à leurs molécules d'acide nucléique cibles respectives, tandis qu'une sonde cadenas hybridée à une sonde de détection liée à une molécule cible non en interaction reste en complexe avec son oligonucléotide de blocage ;
(ii) la réalisation d'une réaction de comblement de brèche et de ligature, pour ainsi générer des produits de ligature circularisés, dans lequel le produit de ligature provenant d'une sonde cadenas en complexe avec son oligonucléotide de blocage comprend une séquence codes-barres provenant de l'oligonucléotide de blocage, de telle sorte que la circularisation des sondes cadenas génère des produits distincts indiquant des molécules cibles en interaction et non en interaction ;
(iii) la détection des produits de circularisation et de leurs taux relatifs, pour ainsi déterminer la proportion de chaque molécule cible interagissant avec les autres, facultativement dans lequel les produits de circularisation sont détectés par séquençage ou qPCR.

13. Procédé selon la revendication 11 ou la revendication 12, dans lequel les deux molécules cibles sont deux protéines, ou une protéine et une molécule d'acide nucléique.

14. Kit de détection d'une molécule d'acide nucléique cible dans un échantillon, comprenant une sonde cadenas et un oligonucléotide de blocage tels que définis dans l'une quelconque des revendications 1 à 4, 8 ou 9.

15. Kit selon la revendication 14, comprenant en outre une sonde de proximité telle que définie dans la revendication 7, facultativement comprenant en outre une paire de sondes cadenas, une paire d'oligonucléotides de blocage et une paire de sondes de proximité telles que définies dans la revendication 12.
